(19)

Europäisches Patentamt

European Patent Office

Office européen des brevets

(11) **EP 4 397 329 A1**

(12) **EUROPEAN PATENT APPLICATION**
published in accordance with Art. 153(4) EPC

(43) Date of publication:
**10.07.2024   Bulletin 2024/28**

(21) Application number: **22865017.2**

(22) Date of filing: **30.08.2022**

(51) International Patent Classification (IPC):
**A61L 27/12** (2006.01)          **A61L 27/56** (2006.01)
**A61F 2/28** (2006.01)

(52) Cooperative Patent Classification (CPC):
**A61F 2/28; A61L 27/12; A61L 27/56**

(86) International application number:
**PCT/KR2022/012954**

(87) International publication number:
**WO 2023/033515 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:   **30.08.2021   KR 20210115120**

(71) Applicants:
• **Sungkwang Medical Foundation**
  **Seoul 06135 (KR)**

• **H&Bio Co., Ltd.**
  **Seongnam-si, Gyeonggi-do 13605 (KR)**

(72) Inventors:
• **SHIM, Jung Hee**
  **Seoul 06004 (KR)**
• **KIM, Yu Ha**
  **Seongnam-si, Gyeonggi-do 13376 (KR)**
• **HAN, Inbo**
  **Seongnam-si, Gyeonggi-do 13597 (KR)**

(74) Representative: **Dr. Schön, Neymeyr & Partner**
**Patentanwälte mbB**
**Bavariaring 26**
**80336 Munich (DE)**

(54) **BONE GRAFT MATERIAL COMPRISING WHITLOCKITE**

(57)    The present application relates to a bone graft material composed of porous whitlockite including nanopores with a size of 100 nm to 1,000 nm, micropores with a size of 1 $\mu$m to 100 $\mu$m, and macropores with a size of 100 $\mu$m to 1,000 $\mu$m. Having pores with various sizes, the bone graft material exhibits the effect of being brought into wide contact with in vivo components and maintaining an appropriate mechanical strength.

FIG. 1

**Description**

[Technical Field]

One embodiment of the present application relates to a bone graft material including whitlockite.
One embodiment of the present application relates to a bone graft material consisting of whitlockite.

[Background Art]

Currently, hydroxyapatite (HAp, $Ca_{10}(PO_4)_6(OH)_2$) and $\beta$-tricalcium phosphate (TCP, $Ca_3(PO_4)_2$) are generally used as bone graft materials that are inserted into the body.
Meanwhile, to form new bone *in vivo,* bone graft materials must have certain levels of physical properties. Specifically, bone graft materials must have physical properties above certain levels in terms of specific surface area, wettability, porosity, and pore size to create a favorable environment for the formation of new bone. In addition, bone graft materials must have certain levels of mechanical strength and thermal strength to secure a space where new bone is formed during the period of new bone formation.
However, currently, hydroxyapatite-based bone graft materials, $\beta$-tricalcium phosphate-based bone graft materials, and composite bone graft materials of hydroxyapatite and $\beta$-tricalcium phosphate, which are commonly used, cannot meet all the requirements for the various physical properties described above, so there are limits to improving the osteogenic ability of bone graft materials implanted in the body.
Therefore, research on bone graft material with improved various physical properties is required.

[Disclosure]

[Technical Problem]

The present application is directed to providing a porous bone graft material consisting of whitlockite.
The present application is directed to providing a porous bone graft material including whitlockite.

[Technical Solution]

One embodiment of the present application provides a porous bone graft material consisting of whitlockite.
Another embodiment of the present application provides a porous bone graft material including whitlockite.
Still another embodiment of the present application provides a bone graft material with improved osteogenic ability and/or bone regenerating ability.
Yet another embodiment of the present application provides a porous bone graft material consisting of whitlockite, which includes the following: nanopores with a size of 100 nm to 1,000 nm; micropores with a size of 1 $\mu$m to 100 $\mu$m; and macropores with a size of 100 $\mu$m to 1000 $\mu$m.
In a specific embodiment, the volume formed by nanopores with a size of 100 nm to 1000 nm per unit mass of the bone graft material may be 0.15 to 0.5 mL/g.
In a specific embodiment, the volume formed by micropores with a size of 1 $\mu$m to 100 $\mu$m per unit mass of the bone graft material may be 0.06 to 0.35 mL/g.
In a specific embodiment, the volume formed by macropores with a size of 100 $\mu$m to 1000 $\mu$m per unit mass of the bone graft material may be 0.7 to 1.6 mL/g.
In a specific embodiment, the volume formed by nanopores with a size of 100 nm to 1000 nm per unit mass of the bone graft material may be 0.15 to 0.5 mL/g, the volume formed by micropores with a size of 1 to 100 $\mu$m per unit mass of the bone graft material may be 0.06 to 0.35 mL/g, and the volume formed by macropores with a size of 100 to 1000 $\mu$m per unit mass of the bone graft material may be 0.7 to 1.6 mL/g.
In a specific embodiment, the vol% of macropores with a size of 100 to 1000 $\mu$m may be 50% to 90%, and may be calculated by the following equation:

$$a\,(\%) = 100 \times \frac{\text{volume formed by macropores per unit mass of whitlockite bone graft material}}{\text{volume formed by all pores per unit mass of whitlockite bone graft material}}$$

Here, a is the vol% of macropores with a size of 100 to 1000 $\mu$m.
In a specific embodiment, the vol% of micropores with a size of 1 to 100 $\mu$m may be 4.5% to 25%, and may be calculated by the following equation:

2

$$b\ (\%) = 100 \times \frac{\text{volume formed by micropores per unit mass of whitlockite bone graft material}}{\text{volume formed by all pores per unit mass of whitlockite bone graft material}}$$

Here, b is the vol% of micropores with a size of 1 to 100 $\mu$m.

In a specific embodiment, the vol% of nanopores with a size of 100 nm to 1000 nm may be 8% to 30%, and may be calculated by the following equation:

$$c\ (\%) = 100 \times \frac{\text{volume formed by nanopores per unit mass of whitlockite bone graft material}}{\text{volume formed by all pores per unit mass of whitlockite bone graft material}}$$

Here, c is the vol% of nanopores with a size of 100 nm to 1000 nm.

In a specific embodiment, the vol% of macropores with a size of 100 to 1000 $\mu$m may be 50% to 90%, the vol% of micropores with a size of 1 to 100 $\mu$m may be 4.5% to 25%, and the vol% of nanopores with a size of 100 nm to 1000 nm may be 8% to 30%. Here, the vol% of macropores with a size of 100 to 1000 $\mu$m, the vol% of micropores with a size of 1 to 100 $\mu$m, and the vol% of nanopores with a size of 100 nm to 1000 nm may be calculated by the following equations, respectively:

$$a\ (\%) = 100 \times \frac{\text{volume formed by macropores per unit mass of whitlockite bone graft material}}{\text{volume formed by all pores per unit mass of whitlockite bone graft material}}$$

$$b\ (\%) = 100 \times \frac{\text{volume formed by micropores per unit mass of whitlockite bone graft material}}{\text{volume formed by all pores per unit mass of whitlockite bone graft material}}$$

$$c\ (\%) = 100 \times \frac{\text{volume formed by nanopores per unit mass of whitlockite bone graft material}}{\text{volume formed by all pores per unit mass of whitlockite bone graft material}}$$

Here, a is the vol% of macropores with a size of 100 to 1000 $\mu$m, b is the vol% of micropores with a size of 1 to 100 $\mu$m, and c is the vol% of nanopores with a size of 100 nm to 1000 nm.

In a specific embodiment, the bone graft material may have a porosity of 60% to 95%.

In a specific embodiment, the bone graft material may have a compressive strength of 500 Kpa to 900 KPa.

In a specific embodiment, the bone graft material may have a specific surface area of 5 $m^2$/g to 6 $m^2$/g.

In a specific embodiment, here, the specific surface area may be analyzed by a Brunauer-Emmett-Teller method.

In a specific embodiment, the bone graft material may have a property of absorbing 2.0 g or more of water per unit mass.

[Advantageous Effects]

According to an embodiment of the present application, a bone graft material with improved osteogenic ability and/or bone regenerating ability can be provided.

The effects of the invention according to the present application are not limited to the above-described effect, and effects that are not mentioned can be clearly understood by those of ordinary skill in the art from the present specification and the accompanying drawings.

[Description of Drawings]

FIG. 1 shows the XRD data of a whitlockite bone graft material (Sample 1) according to an embodiment of the present application.

FIG. 2 shows the XRD data of bone graft materials obtained according to Comparative Examples.

FIG. 3 shows the SEM analysis results of a whitlockite bone graft material (Sample 1-3) according to an embodiment of the present application.

FIGS. 4 and 5 show the SEM analysis results of bone graft materials obtained according to Comparative Examples 1, 2, 3, and 4.

FIG. 6 shows the SEM analysis results of a bone graft material obtained according to Comparative Example 5.

FIG. 7 shows the SEM analysis results of a bone graft material obtained according to Comparative Example 6.

FIG. 8 shows the porosity analysis results of whitlockite bone graft materials (Sample 1-2 to Sample 1-4) according to an embodiment of the present application.

FIG. 9 shows the pore distribution analysis results of a whitlockite bone graft material (Sample 1-2) according to an embodiment of the present application. In FIG. 9, (1) shows the first measurement for Sample 1-2, and (2) shows the second measurement for Sample 1-2.

FIG. 10 shows the pore distribution analysis results of a whitlockite bone graft material (Sample 1-3) according to an embodiment of the present application.

FIG. 11 shows the pore distribution analysis results of a whitlockite bone graft material (Sample 1-4) according to an embodiment of the present application.

FIG. 12 shows the porosity analysis results of bone graft materials obtained according to Comparative Examples 3 to 5.

FIG. 13 shows the pore distribution analysis results of bone graft materials obtained according to Comparative Example 3 and Comparative Example 4, respectively.

FIG. 14 shows the pore distribution analysis results of bone graft materials obtained according to Comparative Example 5 and Comparative Example 6, respectively.

FIG. 15 shows the specific surface area analysis results of a whitlockite bone graft material (Sample 1-2) according to an embodiment of the present application and bone graft materials (Comparative Examples 3 and 4) obtained according to Comparative Examples.

FIG. 16 shows the wettability analysis results of a whitlockite bone graft material (Sample 1-1) according to an embodiment of the present application and bone graft materials (Comparative Examples 1 and 2) obtained according to Comparative Examples, respectively.

FIG. 17 shows the compressive strength analysis results of a whitlockite bone graft material (Sample 1) according to an embodiment of the present application and bone graft materials (Comparative Examples 1 and 2) obtained according to Comparative Examples.

FIG. 18 shows the thermogravimetric analysis results of a whitlockite bone graft material (Sample 1-2) according to an embodiment and bone graft materials (Comparative Examples 3 and 4) obtained according to Comparative Examples.

FIG. 19 shows the ion release analysis results of a whitlockite bone graft material (Sample 1-2) according to an embodiment of the present application and bone graft materials (Comparative Examples 3 and 4) obtained according to Comparative Examples.

FIG. 20 shows the osteogenic ability analysis results of whitlockite bone graft materials (Sample 1-1 and Sample 1-2) according to an embodiment of the present application and bone graft materials (Comparative Examples 1 to 4) obtained according to Comparative Example. Specifically, FIG. 20 shows micro-CT images acquired through micro-CT analysis.

FIG. 21 shows the osteogenic ability analysis results of whitlockite bone graft materials (Sample 1-1 and Sample 1-2) according to an embodiment of the present application and bone graft materials (Comparative Examples 1 to 4) obtained according to Comparative Examples. Specifically, FIG. 21 shows graphs and tables illustrating the NV and TV of a control and experimental groups measured by analyzing micro-CT.

FIG. 22 shows the osteogenic ability analysis results of whitlockite bone graft materials (Sample 1-1 and Sample 1-2) according to an embodiment of the present application and bone graft materials (Comparative Examples 1 to 4) obtained according to Comparative Examples. Specifically, FIG. 22 shows graphs and tables illustrating the NV and TV of a control and experimental groups measured through H&E staining.

FIG. 23 shows the osteogenic ability analysis results of whitlockite bone graft materials (Sample 1-1 and Sample 1-2) according to an embodiment of the present application and bone graft materials (Comparative Examples 1 to 4) obtained according to Comparative Examples. Specifically, FIG. 23 shows graphs and tables illustrating the NV and TV of a control and experimental groups measured through Masson's Trichrome staining.

FIG. 24 shows the osteogenic ability analysis results of whitlockite bone graft materials (Sample 1-1 and Sample 1-2) according to an embodiment of the present application and bone graft materials (Comparative Examples 1 to 4) obtained according to Comparative Examples. Specifically, FIG. 24 shows graphs and tables illustrating the relative proportions of biomarkers associated with osteogenesis measured through immunohistochemistry.

FIG. 25 shows the analysis results of the cell adhesion ability of a whitlockite bone graft material (Sample 1-4) according to an embodiment of the present application and bone graft materials (Comparative Examples 5 and 6) obtained according to Comparative Examples.

[Modes of the Invention]

The above-described objects, feature, and advantages of the present invention will become more apparent through the following detailed description in conjunction with the accompanying drawings. However, the present invention may have various changes and various embodiments, and specific embodiments will be illustrated in the drawings and described in detail.

In the description of the present invention, when it is determined that a detailed description of a known function or configuration related to the present invention may unnecessarily obscure the gist of the present invention, the detailed description will be omitted. In addition, numbers (e.g., first, second, etc.) used in the description of the specification are merely identifiers for distinguishing one component from another component.

**Definition of terms**

Unless defined otherwise, all technical and scientific terms used in the specification have the same meanings as commonly understood by one of ordinary skill in the art to which the present invention belongs. All publications, patent applications, patents and other references mentioned in the specification are incorporated by reference in their entirety.

The term "approximately" used herein means approximately a certain quantity, and an amount, level, value, number, frequency, percentage, dimension, size, quantity, weight, or length that varies by 30, 25, 20, 25, 10, 9, 8, 7, 6, 5, 4, 3, 2, or 1% with respect to the reference amount, level, value, number, frequency, percentage, dimension, size, quantity, weight, or length.

**Whitlockite bone graft materials**

*Whitlockite*

Whitlockite ($Ca_{18}Mg_2(HPO_4)_2(PO_4)_{12}$) is calcium phosphate containing magnesium atoms, and plays an important role in the growth or development of hard tissue as described above. Whitlockite and a conventional method of preparing whitlockite are disclosed in the literature 'Phase transformation from hydroxyapatite to the secondary bone mineral, whitlockite' (Jang, Hae Lin et al., "Phase transformation from hydroxyapatite to the secondary bone mineral, whitlockite." Journal of Materials Chemistry B 3.7 (2015): 1342-1349.) and PCT Application No. WO2021/080348 (Publication No.) in detail.

*Bone graft material*

A bone graft material is implanted, processed, or added into a living body and performs the function of promoting the formation of new bone or bone regeneration. For example, a bone graft material may be added, treated, or implanted at or near a site of a fracture or bone defect to promote new bone formation or bone regeneration.

*Characteristics required for bone graft material*

To promote the formation of new bone *in vivo,* a bone graft material preferably has the following characteristics.

In order to attach more cells to the bone graft material, the bone graft material preferably has nanopores, macropores, and macropores, which have sizes ranging from hundreds of nanometers to hundreds of micrometers as many as possible.

It is preferable that wettability is high so that blood can sufficiently move through the bone graft material. Particularly, it is preferable to have a large number of nanopores with a size of hundreds of nanometers so that blood can sufficiently move through the bone graft material.

To allow cells to enter into or adhere well to the bone graft material, it is preferable that the bone graft material has many nanopores, micropores, and/or macropores with a size of hundreds of nanometers to hundreds of micrometers.

The bone graft material preferably has a large area in contact with blood.

The bone graft material preferably has physical properties that allow them to absorb a large amount of blood per unit mass.

The bone graft material needs to maintain its shape without being damaged by the surrounding environment, such as heat and an external force, during the period in which new bone is formed *in vivo,* and to this end, it is preferable to have mechanical or thermal strength a above certain level.

The bone graft material must have pores of various sizes and maintain appropriate mechanical strength to allow cells associated with osteogenic differentiation and/or osteogenesis to be attached or move, to absorb enough blood and allow enough blood to move, to smoothly grow blood vessels, and to widen a contact surface with a component in the body, such as a contact surface with blood. These characteristics may be determined by the components constituting the bone graft material and the porous structure of the bone graft material.

*Overview of whitlockite bone graft material*

The present application provides a whitlockite bone graft material.
In one embodiment, the whitlockite bone graft material may be a bone graft material including whitlockite.
In one embodiment, the whitlockite bone graft material may be a bone graft material consisting of whitlockite.
In one embodiment, the whitlockite bone graft material may have a porous structure. Here, the porous structure may be used as a meaning encompassing all structures in which pores of any size are formed.
Hereinafter, the whitlockite bone graft material is described in detail.

**Whitlockite bone graft material 1: Bone graft material including whitlockite**

In one embodiment, the whitlockite bone graft material may be a bone graft material including whitlockite. For example, the whitlockite bone graft material of the present application may include whitlockite, and further include calcium phosphate particles including at least one of a trace amount of hydroxyapatite, β-TCP, and a combination thereof. Here, the trace amount may mean a mass ratio of approximately 1% to 15%.

**Whitlockite bone graft material 2: Bone graft material consisting of whitlockite**

In one embodiment, the whitlockite bone graft material may be a bone graft material consisting of whitlockite. In one embodiment, the whitlockite bone graft material may be a bone graft material consisting of whitlockite granules. In one example, the bone graft material according to an embodiment of the present application may consist of 100% whitlockite.

**Porous structure of whitlockite bone graft material**

*Overview of porous structure of whitlockite bone graft material*

The whitlockite bone graft material of the present application has a porous structure. Here, the porous structure may be used as a meaning encompassing all structure having pores of any size. For example, the bone graft material including whitlockite has a porous structure. In another example, the bone graft material consisting of whitlockite has a porous structure.
In one embodiment, the whitlockite bone graft material of the present application may include pores. In a specific embodiment, the whitlockite bone graft material of the present application may include pores with a size of approximately 100 nm to 1000 $\mu$m.
In one embodiment, the whitlockite bone graft material of the present application may include any one or more of nanopores, micropores, and macropores.
Here, the nanopore refers to a pore with a size of 1000 nm or less, and preferably, a pore with a size of 100 nm to 1000 nm. The micropore refers to a pore with a size of approximately 1 to 100 $\mu$m. The macropore refers to a pore with a size of 100 $\mu$m or more, and preferably, a pore with a size of approximately 100 to 1000 $\mu$m.
Hereinafter, pores included in the whitlockite bone graft material will be described in detail.

*Types of pores included in whitlockite bone graft material*

As described above, the whitlockite bone graft material may have a porous structure, and may include any one or more of nanopores, micropores, and macropores.
In one embodiment, the whitlockite bone graft material may include nanopores. In a specific embodiment, the whitlockite bone graft material may include nanopores with a size of approximately 100 nm to 1000 nm.
Whitlockite releases magnesium ions and calcium ions. Magnesium ions and calcium ions promote osteogenesis and/or osteogenic differentiation, or promote the activity of cells associated with osteogenesis and/or osteogenic differentiation. Accordingly, as the amount of magnesium ions and calcium ions, which are released from whitlockite, increases, and/or more cells are attached to whitlockite, osteogenesis and/or osteogenic differentiation is promoted. When nanopores are present, since the roughness of the surface of the bone graft material tends to increase, it may be easy for cells involved in osteogenesis and/or osteogenic differentiation to attach to the bone graft material. When more cells are attached by nanopores, magnesium ions and calcium ions may be delivered more effectively to more cells. In addition, when nanopores are present, the surface area of the bone graft material increases, thereby increasing the amount of magnesium ions and calcium ions, which are released from the whitlockite bone graft material. Further, when nanopores are present, due to the capillary phenomenon caused by small pores such as nanopores, the flow of a body fluid, such as blood, may be promoted in a space where the bone graft material is implanted. Accordingly, osteogenesis or osteogenic differentiation in the surrounding tissue of the implanted area may be promoted. Furthermore, these effects may be further enhanced as the number of nanopores increases.

In one embodiment, the whitlockite bone graft material may include micropores. In a specific embodiment, the whitlockite bone graft material may include micropores with a size of approximately 1 to 100 $\mu$m. When micropores are present, approximately 10 to 30 $\mu$m-sized cells involved in osteogenesis and/or osteogenic differentiation may be attached to a bone graft material having micropores, and a space that is advantageous for growth may be secured. Further, these effects may be more enhanced as the number of micropores increases.

In one embodiment, the whitlockite bone graft material may include macropores. In a specific embodiment, the whitlockite bone graft material may include macropores with a size of approximately 100 to 1000 $\mu$m. When macropores are present, the movement of a body fluid such as blood and cells involved in osteogenesis and/or osteogenic differentiation may be facilitated, thereby promoting osteogenesis and/or osteogenic differentiation. Furthermore, when macropores are present, a space where blood vessels can smoothly grow may be secured, thereby promoting osteogenesis and/or osteogenic differentiation. Moreover, these effects may be further enhanced as the number of macropores increases.

In one embodiment, the whitlockite bone graft material may include nanopores, micropores, and macropores. In a specific embodiment, the whitlockite bone graft material may include nanopores with a size of approximately 100 nm to 1000 nm, micropores with a size of approximately 1 to 100 $\mu$m, and macropores with a size of approximately 100 to 1000 $\mu$m.

In one embodiment, the whitlockite bone graft material may include one or more pores selected from the follows: pores with a size of 1 to 10 nm, pores with a size of 10 to 20 nm, pores with a size of 20 to 30 nm, pores with a size of 30 to 40 nm, pores with a size of 40 to 50 nm, pores with a size of 50 to 60 nm, pores with a size of 60 to 70 nm, pores with a size of 70 to 80 nm, pores with a size of 80 to 90 nm, pores with a size of 90 to 100 nm, pores with a size of 100 to 200 nm, pores with a size of 200 to 300 nm, pores with a size of 300 to 400 nm, pores with a size of 400 to 500 nm, pores with a size of 500 to 600 nm, pores with a size of 600 to 700 nm, pores with a size of 700 to 800 nm, pores with a size of 800 to 900 nm, pores with a size of 900 to 1000 nm, pores with a size of 1 to 10 $\mu$m, pores with a size of 10 to 20 $\mu$m, pores with a size of 20 to 30 $\mu$m, pores with a size of 30 to 40 $\mu$m, pores with a size of 40 to 50 $\mu$m, pores with a size of 50 to 60 $\mu$m, pores with a size of 60 to 70 $\mu$m, pores with a size of 70 to 80 $\mu$m, pores with a size of 80 to 90 $\mu$m, pores with a size of 90 to 100 $\mu$m, pores with a size of 100 to 200 $\mu$m, pores with a size of 200 to 300 $\mu$m, pores with a size of 300 to 400 $\mu$m, pores with a size of 400 to 500 $\mu$m, pores with a size of 500 to 600 $\mu$m, pores with a size of 600 to 700 $\mu$m, pores with a size of 700 to 800 $\mu$m, pores with a size of 800 to 900 $\mu$m, pores with a size of 900 to 1000 $\mu$m, pores with a size of 1000 to 2000 $\mu$m, pores with a size of 2000 to 3000 $\mu$m, pores with a size of 3000 to 4000 $\mu$m, and pores with a size of 4000 to 5000 $\mu$m.

*Volume formed by pores in whitlockite bone graft material*

The whitlockite bone graft material of the present application has a porous structure. That is, the whitlockite bone graft material of the present application includes various sizes of pores. In one embodiment, a volume (mL/g) formed by pores per unit mass of the whitlockite bone graft material may be any one selected from approximately 1, 1.05, 1.1, 1.15, 1.2, 1.25, 1.3, 1.35, 1.4, 1.45, 1.5, 1.55, 1.6, 1.65, 1.7, 1.75, 1.8, 1.85, 1.9, 1.95, 2, 2.05, 2.1, 2.15, 2.2, 2.25, 2.3, 2.35, 2.4, 2.45, 2.5, 2.6, 2.7, 2.8, 2.9, and 3.0 mL/g, or may be in a range set by selecting two of these values. For example, the volume (mL/g) formed by pores per unit mass of the whitlockite bone graft material may be 1 to 2.5 mL/g. In a specific embodiment, the volume formed by pores per unit mass of the whitlockite bone graft material may be 1.2 to 2.2 mL/g. In a specific embodiment, the volume formed by pores per unit mass of the whitlockite bone graft material may be 1.35 to 2.1 mL/g.

**Distribution of pores included in whitlockite bone graft material**

*Analysis of pore distribution included in whitlockite bone graft material*

In one embodiment, the pore distribution in the whitlockite bone graft material may be analyzed. Here, the analysis of the pore distribution refers to analysis, measurement, or calculation with respect to pore type and pore size distribution. Through the analysis of the pore distribution, the volume or area formed by pores of a certain pore type or with a certain pore size may be analyzed. For example, through the analysis of the pore distribution, the volume or area formed by macropores may be analyzed, measured, or calculated. In another example, through the analysis of the pore distribution, the volume or area formed by micropores may be analyzed, measured, or calculated. In still another example, through the analysis of the pore distribution, the volume or area formed by nanopores may be analyzed, measured, or calculated. In one embodiment, a pore size may be a pore diameter.

In one embodiment, the pore distribution may be analyzed by a mercury porosimeter.

*Volume formed by macropores*

As described above, the whitlockite bone graft material may include macropores. In one embodiment, the macropores may have a size (e.g., diameter) of 100 $\mu$m or more. In one embodiment, the macropores may have a size of 100 to 1000 $\mu$m. In one embodiment, the volume of pores formed by macropores per unit mass (mL/g) of the whitlockite bone graft material may be any one selected from approximately 0.5, 0.55, 0.6, 0.65, 0.7, 0.75, 0.8, 0.85, 0.9, 0.95, 1, 1.05, 1.1, 1.15, 1.2, 1.25, 1.3, 1.35, 1.4, 1.45, 1.5, 1.55, 1.6, 1.65, 1.7, 1.75, 1.8, 1.85, 1.9, and 2 mL/g, and may be in a range set by selecting two of these values. For example, the volume of pores formed by macropores per unit mass (mL/g) of the whitlockite bone graft material may be approximately 0.7 to 1.7 mL/g. In a specific embodiment, the volume of pores formed by macropores per unit mass (mL/g) of the whitlockite bone graft material may be approximately 0.8 to 1.55 mL/g.

The macropore distribution in the whitlockite bone graft material may be analyzed as follows.

The vol% of the macropores included in whitlockite, which is information on the volume formed by macropores included in the whitlockite bone graft material may be calculated by following equation:

$$a\,(\%) = 100 \times \frac{\text{volume formed by macropores included in whitlockite bone graft material}}{\text{volume formed by all pores included in whitlockite bone graft material}},$$

or

$$a\,(\%) = 100 \times \frac{\text{volume formed by macropores per unit mass of whitlockite bone graft material}}{\text{volume formed by all pores per unit mass of whitlockite bone graft material}},$$

here, a is the vol% of macropores.

In one embodiment, the vol% of macropores may be approximately 50 to 90%.

In one embodiment, the vol% of macropores may be any one selected from approximately 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, and 90%, or may be in a range set by selecting two of these values. For example, the vol% of macropores may be approximately 55 to 80%. In a specific embodiment, the vol% of macropores may be approximately 60 to 75%. In a specific embodiment, the volume of macropores may be approximately 61% to 75%.

In one embodiment, the volume formed by macropores included in the whitlockite bone graft material may be analyzed, measured, or calculated by a mercury porosimeter. In one embodiment, the volume formed by all pores in the whitlockite bone graft material may be analyzed, measured, or calculated by a mercury porosimeter.

*Volume formed by micropores*

As described above, the whitlockite bone graft material may include micropores. In one embodiment, the micropores may have a size (e.g., diameter) of 1 to 100 $\mu$m. In one embodiment, the volume (mL/g) of pores formed by micropores per unit mass of the whitlockite bone graft material may be any one selected from approximately 0.05, 0.06, 0.07, 0.08, 0.09, 0.1, 0.12, 0.14, 0.16, 0.18, 0.2, 0.22, 0.24, 0.26, 0.28, 0.3, 0.32, 0.34, 0.36, 0.38, and 0.4 mL/g, or may be in the range set by selecting two of these values. For example, the volume of pores formed by micropores per unit mass (mL/g) of the whitlockite bone graft material may be approximately 0.06 to 0.35 mL/g. In a specific embodiment, the volume of pores formed by micropores per unit mass (mL/g) of the whitlockite bone graft material may be approximately 0.07 to 0.3 mL/g. In a specific embodiment, the volume of pores formed by micropores per unit mass (mL/g) of the whitlockite bone graft material may be approximately 0.1 to 0.35 mL/g. In a specific embodiment, the volume of pores formed by micropores per unit mass (mL/g) of the whitlockite bone graft material may be approximately 0.15 to 0.3 mL/g.

In one embodiment, the whitlockite bone graft material may include micropores with a size of approximately 1 to 10 $\mu$m. In one embodiment, the volume of pores formed by micropores with a size of 1 to 10 $\mu$m per unit mass (mL/g) of the whitlockite bone graft material may be any one selected from approximately 0.01, 0.011, 0.012, 0.013, 0.014, 0.015, 0.016, 0.017, 0.018, 0.019, 0.02, 0.025, 0.03, 0.035, 0.04, 0.045, 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.26, 0.28, and 0.3 mL/g, or may be in the range set by selecting two of these values. For example, the volume of pores formed by micropores with a size of 1 to 10 $\mu$m per

unit mass (mL/g) of the whitlockite bone graft material may be approximately 0.015 to 0.3 mL/g. In a specific embodiment, the volume of pores formed by micropores with a size of 1 to 10 $\mu$m per unit mass (mL/g) of the whitlockite bone graft material may be approximately 0.02 to 0.25 mL/g. In a specific embodiment, the volume of pores formed by micropores with a size of 1 to 10 $\mu$m per unit mass (mL/g) of the whitlockite bone graft material may be approximately 0.08 to 0.25 mL/g. In a specific embodiment, the volume of pores formed by micropores with a size of 1 to 10 $\mu$m per unit mass (mL/g) of the whitlockite bone graft material may be approximately 0.1 to 0.25 mL/g. In a specific embodiment, the volume of pores formed by micropores with a size of 1 to 10 $\mu$m per unit mass (mL/g) of the whitlockite bone graft material may be approximately 0.11 to 0.23 mL/g.

In one embodiment, the whitlockite bone graft material may include micropores with a size of 10 to 100 $\mu$m. In one embodiment, the volume of pores formed by micropores with a size of 10 to 100 $\mu$m per unit mass (mL/g) of the whitlockite bone graft material may be any one selected from approximately 0.03, 0.035, 0.04, 0.045, 0.05, 0.055, 0.06, 0.065, 0.07, 0.075, 0.08, 0.085, 0.09, 0.095, and 0.1 mL/g, or may be in the range set by selecting two of these values. For example, the volume of pores formed by micropores with a size of 10 to 100 $\mu$m per unit mass (mL/g) of the whitlockite bone graft material may be approximately 0.03 to 0.09 mL/g. In a specific embodiment, the volume of pores formed by micropores with a size of 10 to 100 $\mu$m per unit mass (mL/g) of the whitlockite bone graft material may be approximately 0.04 to 0.08 mL/g. In a specific embodiment, the volume of pores formed by micropores with a size of 10 to 100 $\mu$m per unit mass (mL/g) of the whitlockite bone graft material may be approximately 0.05 to 0.07 mL/g.

The distribution of micropores included in the whitlockite bone graft material may be analyzed as follows.

The vol% of micropores included in whitlockite, which is information on the volume formed by micropores included in the whitlockite bone graft material may be calculated by the following equation:

$$b\ (\%) = 100 \times \frac{\text{volume formed by micropores included in whitlockite bone graft material}}{\text{volume formed by all pores included in whitlockite bone graft material}},$$

or

$$b\ (\%) = 100 \times \frac{\text{volume formed by micropores per unit mass of whitlockite bone graft material}}{\text{volume formed by all pores per unit mass of whitlockite bone graft material}},$$

here, b is the vol% of micropores.

In one embodiment, the vol% of micropores may be approximately 4 to 25%.

In one embodiment, the vol% of micropores may be any one selected from approximately 3, 3.2, 3.4, 3.6, 3.8, 4, 4.2, 4.4, 4.6, 4.8, 5, 5.2, 5.4, 5.6, 5.8, 6, 6.2, 6.4, 6.6, 6.8, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 26, 27, 28, 29, and 30%, or may be in the range set by selecting two of these values. For example, the vol% of micropores may be approximately 4 to 25%. In a specific embodiment, the vol% of micropores may be approximately 8 to 22%. In a specific embodiment, the vol% of micropores may be approximately 5 to 20%. In a specific embodiment, the vol% of micropores may be approximately 9 to 20%.

In one embodiment, the whitlockite bone graft material may include micropores with a size of 1 to 10 $\mu$m. In one embodiment, the vol% of micropores with a size of 1 to 10 $\mu$m may be any one selected from approximately 0.8, 0.9, 1, 1.1, 1.15, 1.2, 1.25, 1.3, 1.35, 1.4, 1.45, 1.5, 1.6, 1.7, 1.8, 1.9, 2.0, 2.2, 2.4, 2.6, 2.8, 3, 3.2, 3.4, 3.6, 3.8, 4, 4.5, 5, 5.5, 6, 6.5, 7, 7.5, 8, 8.5, 9, 10, 11, 12, 13, 14, 15, 16, 17, 18, 19, and 20%, or may be in the range set by selecting two of these values. For example, the vol% of micropores with a size of 1 to 10 $\mu$m may be approximately 1.2 to 18%. In a specific embodiment, the vol% of micropores with a size of 1 to 10 $\mu$m may be approximately 1.3 to 17%. In a specific embodiment, the vol% of micropores with a size of 1 to 10 $\mu$m may be approximately 6 to 17%. In a specific embodiment, the vol% of micropores with a size of 1 to 10 $\mu$m may be approximately 6.5 to 16%. Here, the vol% of micropores with a size of 1 to 10 $\mu$m may be calculated by the following equation:

100 x (volume formed by pores with a size of 1 to 10 $\mu$m in whitlockite bone graft material)/(volume formed by all pores in whitlockite bone graft material);

or 100 x (volume formed by pores with a size of 1 to 10 $\mu$m per unit mass of whitlockite bone graft material)/(volume formed by all pores per unit mass of whitlockite bone graft material).

In one embodiment, the whitlockite bone graft material may include micropores with a size of 10 to 100 $\mu$m. In one embodiment, the vol% of the micropores with a size of 10 to 100 $\mu$m may be any one selected from approximately 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.5, 6, 6.5, and 7%, or may be in the range set by selecting two of these values. For example, the vol% of the micropores with a size of 10 to 100 $\mu$m may be approximately 2 to 5%. In a specific embodiment, the vol% of the micropores with a size of 10 to 100 $\mu$m may be approximately 2 to 5%. In a specific embodiment, the vol% of the micropores with a size of 10 to 100 $\mu$m may be approximately 2 to 4.5%. In a specific embodiment, the vol% of the micropores with a size of 10 to 100 $\mu$m may be approximately 2.5 to 3.5%. In a specific embodiment, the vol% of the micropores with a size of 10 to 100 $\mu$m may be approximately 2.8 to 3.4%. Here, the vol% of the micropores with a size of 10 to 100 $\mu$m may be calculated by the following equation:

100 x (volume formed by pores with a size of 10 to 100 $\mu$m in whitlockite bone graft material)/(volume formed by all pores in whitlockite bone graft material);

or 100 x (volume formed by pores with a size of 10 to 100 $\mu$m per unit mass of whitlockite bone graft material)/(volume formed by all pores per unit mass of whitlockite bone graft material).

In one embodiment, the volume formed by micropores included in the whitlockite bone graft material may be analyzed, measured, or calculated by a mercury porosimeter. In one embodiment, the volume formed by all pores in whitlockite bone graft material may be analyzed, measured, or calculated by a mercury porosimeter.

*Volume formed by nanopores*

As described above, the whitlockite bone graft material may include nanopores. In one embodiment, the nanopores may have a size (e.g., diameter) of 1 $\mu$m or less. In one embodiment, the nanopores may have a size of 100 nm to 1 $\mu$m. In one embodiment, the volume of pores formed by nanopores per unit mass (mL/g) of the whitlockite bone graft material may be any one selected from approximately 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.42, 0.44, 0.46, 0.48, 0.5, 0.55, and 0.6 mL/g, or may be in the range set by selecting two of these values. For example, the volume of pores formed by nanopores per unit mass (mL/g) of the whitlockite bone graft material may be approximately 0.15 to 0.5 mL/g. In a specific embodiment, the volume of pores formed by nanopores per unit mass (mL/g) of the whitlockite bone graft material may be approximately 0.2 to 0.5 mL/g. In a specific embodiment, the volume of pores formed by nanopores per unit mass (mL/g) of the whitlockite bone graft material may be approximately 0.2 to 0.4 mL/g. In a specific embodiment, the volume of pores formed by nanopores per unit mass (mL/g) of the whitlockite bone graft material may be approximately 0.25 to 0.35 mL/g.

In one embodiment, the whitlockite bone graft material may include nanopores with a size of 100 to 500 nm. In one embodiment, the volume of pores formed by nanopores with a size of 100 to 500 nm per unit mass (mL/g) of the whitlockite bone graft material may be any one of approximately 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.42, 0.44, 0.46, 0.48, 0.5, 0.55, and 0.6 mL/g. For example, the volume of pores formed by nanopores with a size of 100 to 500 nm per unit mass (mL/g) of the whitlockite bone graft material may be approximately 0.08 to 0.45 mL/g. In a specific embodiment, the volume of pores formed by nanopores with a size of 100 to 500 nm per unit mass (mL/g) of the whitlockite bone graft material may be approximately 0.08 to 0.16 mL/g. In a specific embodiment, the volume of pores formed by nanopores with a size of 100 to 500 nm per unit mass (mL/g) of the whitlockite bone graft material may be approximately 0.1 to 0.13 mL/g.

In one embodiment, the whitlockite bone graft material may include nanopores with a size of 500 nm to 1 $\mu$m. In one embodiment, the volume of pores formed by nanopores with a size of 500 nm to 1 $\mu$m per unit mass (mL/g) of the whitlockite bone graft material may be any one selected from approximately 0.005, 0.006, 0.007, 0.008, 0.009, 0.01, 0.011, 0.012, 0.013, 0.014, 0.015, 0.016, 0.017, 0.018, 0.019, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.3, 0.31, 0.32, 0.33, 0.34, and 0.35 mL/g, or may be in the range set by selecting two of these values. For example, the volume of pores formed by nanopores with a size of 500 nm to 1 $\mu$m per unit mass (mL/g) of the whitlockite bone graft material may be approximately 0.008 to 0.3 mL/g. In a specific embodiment, the volume of pores formed by nanopores with a size of 500 nm to 1 $\mu$m per unit mass (mL/g) of the whitlockite bone graft material may be approximately 0.1 to 0.3 mL/g. In a specific embodiment, the volume of pores formed by nanopores with a size of 500 nm to 1 μm per unit mass (mL/g) of the whitlockite bone graft material may be approximately 0.12 to 0.25 mL/g. In a specific embodiment, the volume of pores formed by nanopores with a size of 500 nm to 1 μm per unit mass (mL/g) of the whitlockite bone graft material may be approximately 0.15 to 0.22 mL/g.

The distribution of nanopores included in the whitlockite bone graft material may be analyzed as follows.

The vol% of the nanopores included in whitlockite, which is information on the volume formed by nanopores included in the whitlockite bone graft material may be calculated by following equation:

$$c\ (\%) = 100 \times \frac{\text{volume formed by nanopores included in whitlockite bone graft material}}{\text{volume formed by all pores included in whitlockite bone graft material}},$$

or

$$c\ (\%) = 100 \times \frac{\text{volume formed by nanopores per unit mass of whitlockite bone graft material}}{\text{volume formed by all pores per unit mass of whitlockite bone graft material}},$$

here, c is the vol% of nanopores.

In one embodiment, the vol% of nanopores may be 8 to 30%.

In one embodiment, the vol% of nanopores may be any one selected from approximately 8, 8.2, 8.4, 8.6, 8.8, 9, 9.2, 9.4, 9.6, 9.8, 10, 10.2, 10.4, 10.6, 10.8, 11, 11.2, 11.4, 11.6, 11.8, 12, 12.2, 12.4, 12.6, 12.8, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, and 30%, or may be in the range set by selecting two of these values. For example, the vol% of nanopores may be approximately 10 to 28%. In a specific embodiment, the vol% of nanopores may be approximately 12 to 27%. In a specific embodiment, the vol% of nanopores may be approximately 12 to 23%. In a specific embodiment, the vol% of nanopores may be approximately 12 to 22%.

In one embodiment, the whitlockite bone graft material may include nanopores with a size of 100 to 500 nm. In one embodiment, the vol% of nanopores with a size of 100 to 500 nm may be any one selected from approximately 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.2, 10.4, 10.6, 10.8, 11, 11.5, 12, 12.5, 13, 13.5, 14, 14.5, 15, 15.5, 16, 16.5, 17, 17.5, 18, 18.4, 19, 19.5, 20, 20.5, 21, 21.5, 22, 22.5, 23, 23.5, 24, 24.5, 25, 25.5, 26, 26.5, 27, 27.5, 28, 28.5, 29, 29.5, and 30%, or may be in the range set by selecting two of these values. For example, the vol% of nanopores with a size of 100 to 500 nm may be approximately 4 to 28%. In a specific embodiment, the vol% of nanopores with a size of 100 to 500 nm may be approximately 4 to 12%. In a specific embodiment, the vol% of nanopores with a size of 100 to 500 nm may be approximately 4.5 to 9%. In a specific embodiment, the vol% of nanopores with a size of 100 to 500 nm may be approximately 4.8 to 8.2%. Here, the vol% of nanopores with a size of 100 to 500 nm may be calculated by the following equation:

100 x (volume formed by pores with a size of 100 to 500 nm in whitlockite bone graft material)/(volume formed by all pores in whithlockite bone graft material);

or 100 x (volume formed by pores with a size of 100 to 500 nm per unit mass of whitlockite bone graft material)/(volume formed by all pores per unit mass of whitlockite bone graft material).

In one embodiment, the whitlockite bone graft material may include nanopores with a size of 500 nm to 1 μm. In one embodiment, the vol% of nanopores with a size of 500 nm to 1 μm may be any one selected from approximately 0.4, 0.45, 0.5, 0.55, 0.6, 0.65, 0.7, 0.75, 0.8, 0.85, 0.9, 0.95, 1, 1.05, 1.1, 1.15, 1.2, 1.25, 1.3, 1.35, 1.4, 1.45, 1.5, 1.55, 1.6, 1.65, 1.7, 1.75, 1.8, 1.85, 1.9, 2, 2.1, 2.2, 2.3, 2.4, 2.5, 2.6, 2.7, 2.8, 2.9, 3, 3.2, 3.4, 3.6, 3.8, 4, 4.2, 4.4, 4.6, 4.8, 5, 5.5, 6, 6.2, 6.4, 6.6, 6.8, 7, 7.2, 7.4, 7.6, 7.8, 8, 8.5, 9, 9.5, 10, 10.5, 11, 11.5, 12, 12.5, 13, 13.5, 14, 14.5, 15, 15.5, 16, 16.5, 17, 18, 19, and 20%, or may be in the range set by selecting two of these values. For example, the vol% of nanopores with a size of 500 nm to 1 μm may be approximately 0.5 to 15%. In a specific embodiment, the vol% of nanopores with a size of 500 nm to 1 μm may be approximately 6.6 to 15%. In a specific embodiment, the vol% of nanopores with a size of 500 nm to 1 μm may be approximately 7 to 14%. In a specific embodiment, the vol% of nanopores with a size of 500 nm to 1 $\mu$m may be approximately 7.2 to 13.5%. Here, the vol% of nanopores with a size of 500 nm to 1 $\mu$m may be calculated by the following equation:

100 x (volume formed by pores with a size of 500 nm to 1 $\mu$m in whitlokite bone graft material)/(volume formed by all pores in whithlockite bone graft material);

 or 100 x (volume formed by pores with a size of 500 nm to 1 $\mu$m per unit mass of whitlockite bone graft material)/(volume formed by all pores per unit mass of whitlockite bone graft material).

In one embodiment, the volume formed by nanopores included in the whitlockite bone graft material may be analyzed, measured, or calculated by a mercury porosimeter. In one embodiment, volume formed by all pores in the whitlockite bone graft material may be analyzed, measured, or calculated by a mercury porosimeter.

*Volume formed by nano-micropores*

In one embodiment, the whitlockite bone graft material may include pores with a size of 100 nm to 10 $\mu$m. In one embodiment, the volume formed by pores with a size of 100 nm to 10 $\mu$m per unit mass (mL/g) of the whitlockite bone graft material may be any one selected from approximately 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.52, 0.54, 0.56, 0.58, 0.6, 0.62, 0.64, 0.66, 0.68, and 0.7, or may be in the range set by selecting two of these values. For example, the volume formed by pores with a size of 100 nm to 10 $\mu$m per unit mass (mL/g) of the whitlockite bone graft material may be approximately 0.3 to 0.6 mL/g. In a specific embodiment, the volume formed by pores with a size of 100 nm to 10 $\mu$m per unit mass (mL/g) of the whitlockite bone graft material may be approximately 0.4 to 0.6 mL/g. In a specific embodiment, the volume formed by pores with a size of 100 nm to 10 $\mu$m per unit mass (mL/g) of the whitlockite bone graft material may be approximately 0.4 to 0.55 mL/g. In a specific embodiment, the volume formed by pores with a size of 100 nm to 10 $\mu$m per unit mass (mL/g) of the whitlockite bone graft material may be approximately 0.4 to 0.5 mL/g. In a specific embodiment, the volume formed by pores with a size of 100 nm to 10 $\mu$m per unit mass (mL/g) of the whitlockite bone graft material may be approximately 0.45 to 0.49 mL/g.
In one embodiment, the vol% of pores with a size of 100 nm to 10 $\mu$m may be any one selected from approximately 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 37, 38, 39, 40, 41, 42, 43, 44, and 45%, or may be in the range set by selecting two of these values. For example, the vol% of pores with a size of 100 nm to 10 $\mu$m may be approximately 20 to 40%. In a specific embodiment, the vol% of pores with a size of 100 nm to 10 $\mu$m may be approximately 20 to 38%. In a specific embodiment, the vol% of pores with a size of 100 nm to 10 $\mu$m may be approximately 22 to 36%. Here, the vol% of pores with a size of 100 nm to 10 $\mu$m may be calculated by the following equation:

100 x (volume formed by pores with a size of 100 nm to 10 $\mu$m in whitlockite bone graft material)/(volume formed by all pores in whitlockite bone graft material);

 or 100 x (volume formed by pores with a size of 100 nm to 10 $\mu$m per unit mass of whitlockite bone graft material)/(volume formed by all pores per unit mass of whitlockite bone graft material).

In one embodiment, the whitlockite bone graft material may include pores with a size of 100 nm to 100 $\mu$m. In one embodiment, the volume formed by pores with a size of 100 nm to 100 $\mu$m per unit mass (mL/g) of the whitlockite bone graft material may be any one selected from approximately 0.3, 0.31, 0.32, 0.33, 0.34, 0.35, 0.36, 0.37, 0.38, 0.39, 0.4, 0.42, 0.44, 0.46, 0.48, 0.5, 0.52, 0.54, 0.56, 0.58, 0.6, 0.62, 0.64, 0.66, 0.68, 0.7, 0.75, and 0.8 mL/g, or may be in the range set by selecting two of these values. For example, the volume formed by pores with a size of 100 nm to 100 $\mu$m per unit mass (mL/g) of the whitlockite bone graft material may be approximately 0.3 to 0.7 mL/g. In a specific embodiment, the volume formed by pores with a size of 100 nm to 100 $\mu$m per unit mass (mL/g) of the whitlockite bone graft material may be approximately 0.4 to 0.6 mL/g. In a specific embodiment, the volume formed by pores with a size of 100 nm to 100 $\mu$m per unit mass (mL/g) of the whitlockite bone graft material may be approximately 0.45 to 0.55 mL/g.

In one embodiment, the vol% of pores with a size of 100 nm to 100 $\mu$m may be any one selected from 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 29.5, 30, 31.5, 32, 32.5, 33, 33.5, 34, 34.5, 35, 35.5, 36, 36.5, 37, 37.5, 38, 38.5, 39, 39.5, 40, 41, 42, 43, 44, 45, 46, 47, 48, 49, and 50%, or may be in the range set by selecting two of these values. For example, the vol% of pores with a size of 100 nm to 100 $\mu$m may be approximately 20 to 45%. In a specific embodiment, the vol% of pores with a size of 100 nm to 100 $\mu$m may be approximately 24 to 42%. In a specific embodiment, the vol% of pores with a size of 100 nm to 100 $\mu$m may be approximately 26 to 39%. Here, the vol% of pores with a size of 100 nm to 100 $\mu$m may be calculated by the following equation:

100 x (volume formed by pores with a size of 100 nm to 100 $\mu$m in whitlockite bone graft material)/(volume formed by all pores in whitlockite bone graft material);

or 100 x (volume formed by pores with a size of 100 nm to 100 $\mu$m per unit mass of whitlockite bone graft material)/(volume formed by all pores per unit mass of whitlockite bone graft material).

*Volume ratio of macropores and micropores*

In one embodiment, the volume ratio (a/b) of macropores and micropores included in the whitlockite bone graft material may be 1.5 to 10.

In one embodiment, the volume ratio (a/b) of macropores and micropores included in the whitlockite bone graft material may be any one selected from approximately 1.5, 2, 2.5, 3, 3.5, 4, 4.5, 5, 5.5, 6, 6.5, 7, 7.5, 8, 8.5, 9, 9.5, and 10, or may be in the range set by selecting two of these values. For example, the volume ratio (a/b) of macropores and micropores included in the whitlockite bone graft material may be approximately 2.5 to 6.5. In a specific embodiment, the volume ratio (a/b) of macropores and micropores included in the whitlockite bone graft material may be approximately 3.2 to 6.

*Volume ratio of macropores and nanopores*

In one embodiment, the volume ratio (a/c) of macropores and nanopores included in the bone graft material may be 1.5 to 15.

In one embodiment, the volume ratio (a/c) of macropores and nanopores included in the bone graft material may be any one selected from approximately 1.5, 1.75, 2, 2.25, 2.5, 2.75, 3, 3.25, 3.5, 3.75, 4, 4.25, 4.5, 4.75, 5, 5.25, 5.5, 5.75, 6, 6.25, 6.5, 6.75, 7, 7.25, 7.5, 7.75, 8, 8.5, 9, 9.5, 10, 10.5, 11, 11.5, 12, 12.5, 13, 13.5, 14, 14.5, and 15, or may be in the range set by selecting two of these values. For example, the volume ratio (a/c) of macropores and nanopores included in the bone graft material may be approximately 3 to 13. In a specific embodiment, the volume ratio (a/c) of macropores and nanopores included in the bone graft material may be approximately 3 to 7.25.

*Volume ratio of micropores and nanopores*

In one embodiment, the volume ratio (b/c) of micropores and nanopores included in the whitlockite bone graft material may be 0.1 to 1.5.

In one embodiment, the volume ratio (b/c) of micropores and nanopores included in the whitlockite bone graft material may be any one selected from approximately 0.1, 0.11, 0.12, 0.13, 0.14, 0.15, 0.2, 0.25, 0.3, 0.35, 0.4, 0.45, 0.5, 0.55, 0.6, 0.65, 0.7, 0.75, 0.8, 0.85, 0.9, 0.95, 1, 1.05, 1.1, 1.15, 1.2, 1.25, 1.3, 1.35, 1.4, 1.45, and 1.5, or may be in the range set by selecting two of these values. For example, the volume ratio (b/c) of micropores and nanopores included in the whitlockite bone graft material may be approximately 0.2 to 1.2. In a specific embodiment, the volume ratio (b/c) of micropores and nanopores included in the whitlockite bone graft material may be approximately 0.45 to 1.15.

**Porosity**

Porosity is related to the degree of penetration of blood or cells into the bone graft material. For example, as the porosity is higher, the penetration of blood or cells into the bone graft material is easier, which may be advantageous for new bone formation.

In one embodiment, the whitlockite bone graft material may have a porosity of 50% or more. In one embodiment, the porosity of the whitlockite bone graft material may be one selected from approximately 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, and 95%, or may be in the range set by selecting two of these values. For example, the whitlockite bone graft material may have a porosity of approximately 60 to 95%. In a specific embodiment, the whitlockite bone graft material may have a porosity of approximately 65 to 92%. In a specific embodiment, the whitlockite bone graft material may have a porosity of approximately 68 to 90%. In a specific embodiment, the whitlockite bone graft material may have a porosity of approximately 75 to 90%. In a specific embodiment, the whitlockite bone graft material may have a porosity of approximately 78 to 88%.

**Shape and size of whitlockite bone graft material**

In one embodiment, the whitlockite bone graft material may have a granular form.

In one embodiment, the size of the whitlockite bone graft material may be 0.1 mm or more. In one embodiment, the size of the whitlockite bone graft material may be 0.1 to 20 mm. In one embodiment, the size of the whitlockite bone graft material may be any one selected from 0.5, 0.6, 0.7, 0.8, 0.9, 1, 1.1, 1.2, 1.3, 1.4, 1.5, 1.6, 1.7, 1.8, 1.9, 2, 2.2, 2.4, 2.6, 2.8, 3.0, 3.2, 3.4, 3.6, 3.8, 4, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10, 11, 12, 13, 14, 15, 16, 17, 18, 19, and 20 mm, or may be in the range set by selecting two of these values. In a specific embodiment, the size of the whitlockite bone graft material may be 0.5 to 8 mm. In a specific embodiment, the size of the whitlockite bone graft material may be 0.6 to 1 mm. In a specific embodiment, the size of the whitlockite bone graft material may be 1 to 2 mm. In a specific embodiment, the size of the whitlockite bone graft material may be 1 to 3 mm. In a specific embodiment, the size of the whitlockite bone graft material may be 3 to 6 mm.

**Compressive strength of whitlockite bone graft material**

Compressive strength refers to the property of being able to withstand compressive stress without being damaged. In bone graft materials, compressive strength is an important physical property for securing a space where new bone will be formed during a period in which new bone is formed. The compressive strength may be determined by the components and structure of the bone graft material, and the type and distribution of pores included in the bone graft material.

In one embodiment, the whitlockite bone graft material may have a compressive strength of 500 kilopascal (kPa) or more. For example, the whitlockite bone graft material may have a compressive strength of 500 to 1000 kPa. For example, the whitlockite bone graft material may have a compressive strength of 500 to 900 kPa. For example, the whitlockite bone graft material may have a compressive strength of 550 to 850 kPa. For example, the whitlockite bone graft material may have a compressive strength of 600 to 800 kPa. For example, the whitlockite bone graft material may have a compressive strength of 603 to 780 kPa. For example, the whitlockite bone graft material may have a compressive strength of approximately 691 kPa.

The bone graft material according to an embodiment of the present application may maintain its shape without being destroyed by an external force during a period in which new bone is formed, which may be advantageous in securing a space where new bone is formed.

In addition, the bone graft material according to an embodiment of the present application may be prevented from being destroyed by an external force before implantation in the body. In addition, the destruction of particles is prevented, so handling of the bone graft material may be relatively easy when implanting the bone graft material in the body.

**Specific surface area of whitlockite bone graft material (method of analyzing specific surface area)**

The surface area per unit mass, that is, the specific surface area of a bone graft material directly affects an area in contact with blood or cells in the body, generally, as the specific surface area increases, the area in contact with blood or cells *in vivo* expands, which is known to be advantageous for new bone formation.

In one embodiment, the whitlockite bone graft material may have a specific surface area of 5 $m^2$/g or more. In a specific embodiment, the whitlockite bone graft material may have a specific surface area of 5 to 10 $m^2$/g. In a specific embodiment, the whitlockite bone graft material may have a specific surface area of 5 to 8 $m^2$/g. In a specific embodiment, the whitlockite bone graft material may have a specific surface area of 5 to 6 $m^2$/g.

In one embodiment, the specific surface area of the whitlockite bone graft material may be analyzed by a Brunauer-Emmett-Teller method. For example, the specific surface area of the whitlockite bone graft material may be analyzed by Tristar II 3020 (Micromeritics). In one embodiment, the specific surface area of the whitlockite bone graft material may be analyzed for pores with a size of 2 to 300 nm.

The whitlockite bone graft material according to an embodiment of the present application may have a relatively high specific surface area characteristic. Thus, when implanted in the body, the whitlockite bone graft material has a larger area in contact with blood or cells in the body, which may be advantageous for new bone formation. Here, the larger area in contact with blood means that there is a greater chance of releasing magnesium ions, calcium ions, or phosphorous ions from the bone graft material. Accordingly, as the contact area between the bone graft material and blood increases, ion releasing ability may be maximized.

**Wettability of whitlockite bone graft material**

Wettability is a physical property that refers to the amount of water (or blood) absorbed per unit mass. It is generally known that the greater the amount of water absorbed by the bone graft material per unit mass, the more blood it absorbs, which is advantageous for new bone formation.

In one embodiment, the whitlockite bone graft material may absorb 2.0 g or more of water per unit mass. In one embodiment, the whitlockite bone graft material may absorb 2.2 g or more of water per unit mass. For example, the whitlockite bone graft material may have a physical property of absorbing up to approximately 2.5 g of water.

The whitlockite bone graft material according to an embodiment of the present application may exhibit a characteristic of allowing a relative amount of blood to penetrate into the bone graft material. Accordingly, the whitlockite bone graft material according to an embodiment of the present application may exhibit a beneficial effect on new bone formation.

Additionally, since the whitlockite bone graft material according to an embodiment of the present application may absorb a relatively large amount of highly viscous blood, scattering between particles may be prevented. In addition, as the scattering between particles is prevented, the bone graft material may be more easily manipulated during the procedure of implanting the bone graft material in the body.

**Use of whitlockite bone graft material**

The whitlockite bone graft material according to an embodiment of the present application may have a granular form. In one embodiment, a plurality of granules of the whitlockite bone graft material may be packaged in one container for distribution or use.

The whitlockite bone graft material according to an embodiment of the present application may promote the generation of bone tissue *in vivo.* In one embodiment, the whitlockite bone graft material may be used to replace hard tissue. For example, the whitlockite bone graft material may be used to replace a bone defect area in the skull area. However, this is merely an example, and the whitlockite bone graft material may be used to replace any hard tissue other than the skull, including teeth, vertebrae, etc. In one embodiment, the whitlockite bone graft material may be used to reconstruct hard tissue. For example, the whitlockite bone graft material may be used to reconstruct bone in any part of the spine. However, this is merely an example, and the whitlockite bone graft material may be used to reconstruct any hard tissue including the skull, teeth, etc., other than the spine.

**Effects of whitlockite bone graft material**

As described above, the whitlockite bone graft material according to an embodiment of the present application may include nano-sized pores, have a physical property of a high specific surface area, or have a physical property of absorbing a large amount of water per unit mass. Therefore, according to the whitlockite bone graft material according to an embodiment of the present application, it is advantageous for osteogenesis and osteogenic differentiation-related cells to attach, the area in contact with blood or cells may increase, and the osteogenesis and osteogenic differentiation-related cells may easily penetrate into the bone graft material. Accordingly, according to the whitlockite bone graft material according to an embodiment of the present application, excellent osteogenic ability may be exhibited.

Meanwhile, the whitlockite bone graft material according to an embodiment of the present application may include whitlockite, or consist of whitlockite. Whitlockite, which is a material in which some of the calcium atoms constituting hydroxyapatite are substituted with magnesium atoms, may release magnesium ions into blood in the body. It is known that magnesium ions serve to promote osteogenesis *in vivo.* Therefore, according to the whitlockite bone graft material, osteogenic ability may be improved.

In addition, as described above, the whitlockite bone graft material according to an embodiment of the present application may have relatively high mechanical strength. For example, the whitlockite bone graft material may have a compressive strength that can withstand relatively high compressive stress. Therefore, the whitlockite bone graft material according to an embodiment of the present application maintains its shape without being damaged during the period in which new bone is formed when implanted in the body, which may be more advantageous in securing a space for forming bone.

The above-described effects of the whitlockite bone graft material according to an embodiment of the present application may be deduced or determined by components constituting the whitlockite bone graft material, and particular structures, such as the type and distribution of pores, of the whitlockite bone graft material according to an embodiment of the present invention.

**Embodiments**

Examples of embodiments for the whitlockite bone graft material provided by the present application will be provided below. Meanwhile, the following embodiments merely disclose some examples of various embodiments that can be provided by the present application, so the scope of the content disclosed by the present application should not be limited to the following examples.

A01.

A porous, bone graft material consisting of whitlockite, including:

nanopores with a size of 100 to 1000 nm;
micropores with a size of 1 to 100 $\mu$m; and
macropores with a size of 100 to 1000 $\mu$m.

A02.

A porous, bone graft material including whitlockite, including:

nanopores with a size of 100 to 1000 nm;
micropores with a size of 1 to 100 $\mu$m; and
macropores with a size of 100 to 1000 $\mu$m.

A03.

The bone graft material of any one of A01 and A02, wherein a volume formed by nanopores with the size of 100 to 1000 nm per unit mass of the bone graft material is 0.15 to 0.5 mL/g.

A04.

The bone graft material of A03, wherein the volume formed by nanopores with the size of 100 to 1000 nm per unit mass of the bone graft material is 0.2 to 0.4 mL/g.

A05.

The bone graft material of any one of A01 to A04, wherein the volume formed by micropores with the size of 1 to 100 $\mu$m per unit mass of the bone graft material is 0.06 to 0.35 mL/g.

A06.

The bone graft material of A05, wherein the volume formed by micropores with the size of 1 to 100 $\mu$m per unit mass of the bone graft material is 0.14 to 0.3 mL/g.

A07.

The bone graft material of any one of A01 to A06, wherein the volume formed by macropores with the size of 100 to 1000 $\mu$m per unit mass of the bone graft material is 0.7 to 1.6 mL/g.

A08.

The bone graft material of A07, wherein the volume formed by macropores with the size of 100 to 1000 $\mu$m per unit mass of the bone graft material is 0.8 to 1.6 mL/g.

A09.

The bone graft material of any one of A01 to A08, wherein the volume formed by nanopores with the size of 100 to 1000 nm per unit mass of the bone graft material is 0.15 to 0.5 mL/g,

the volume formed by micropores with the size of 1 to 100 μm per unit mass of the bone graft material is 0.06 to 0.35 mL/g, and
the volume formed by macropores with the size of 100 to 1000 μm per unit mass of the bone graft material is 0.7 to 1.6 mL/g.

A10.

The bone graft material of any one of A01 to A09,

wherein a vol% of the macropores with the size of 100 to 1000 μm is 50% to 90%,
wherein the vol% of the macropores with the size of 100 to 1000 μm is calculated by the following equation:

$$a\ (\%) = 100 \times \frac{\text{volume formed by macropores per unit mass of whitlockite bone graft material}}{\text{volume formed by all pores per unit mass of whitlockite bone graft material}},$$

where a is the vol% of the macropores with a size of 100 to 1000 μm.

A11.

The bone graft material of any one of A01 to A10,

wherein a vol% of the micropores with the size of 1 to 100 μm is 4.5% to 25%,
wherein the vol% of the micropores with the size of 1 to 100 μm is calculated by the following equation:

$$b\ (\%) = 100 \times \frac{\text{volume formed by micropores per unit mass of whitlockite bone graft material}}{\text{volume formed by all pores per unit mass of whitlockite bone graft material}},$$

where b is the vol% of the micropores with the size of 1 to 100 μm.

A12.

The bone graft material of any one of A01 to A11,

wherein a vol% of the nanopores with the size of 100 to 1000 nm is 8% to 30%,
wherein the vol% of the nanopores with the size of 100 to 1000 nm is calculated by the following equation:

$$c\ (\%) = 100 \times \frac{\text{volume formed by nanopores per unit mass of whitlockite bone graft material}}{\text{volume formed by all pores per unit mass of whitlockite bone graft material}},$$

where c is the vol% of the nanopores with a size of 100 to 1000 nm.

A13.

The bone graft material of any one of A01 to A12,

wherein a vol% of the macropores with the size of 100 to 1000 μm is 50% to 90%,
a vol% of the micropores with the size of 1 to 100 μm is 4.5% to 25%, and

a vol% of the nanopores with the size of 100 to 1000 nm is 8% to 30%,
wherein the vol% of the macropores with the size of 100 to 1000 $\mu$m, the vol% of the micropores with the size of 1 to 100 $\mu$m, and the vol% of the nanopores with the size of 100 to 1000 nm are calculated by the following equations, respectively:

$$a\ (\%) = 100 \times \frac{\text{volume formed by macropores per unit mass of whitlockite bone graft material}}{\text{volume formed by all pores per unit mass of whitlockite bone graft material}}$$

$$b\ (\%) = 100 \times \frac{\text{volume formed by micropores per unit mass of whitlockite bone graft material}}{\text{volume formed by all pores per unit mass of whitlockite bone graft material}}$$

$$c\ (\%) = 100 \times \frac{\text{volume formed by nanopores per unit mass of whitlockite bone graft material}}{\text{volume formed by all pores per unit mass of whitlockite bone graft material}}\ ,$$

where a is the vol% of the macropores with a size of 100 to 1000 $\mu$m, b is the vol% of the micropores with a size of 1 to 100 $\mu$m, and c is the vol% of the nanopores with a size of 100 to 1000 nm.

A14.

The bone graft material of A13,
wherein the vol% of the macropores with the size of 100 to 1000 $\mu$m is 60 to 75%.

A15.

The bone graft material of any one of A13 and A14,
wherein the vol% of the micropores with the size of 1 to 100 $\mu$m is 8.5 to 23%.

A16.

The bone graft material of any one of A13 to A15,
wherein the vol% of the nanopores with the size of 100 to 1000 nm is 10 to 25%.

A17.

A porous, bone graft material consisting of whitlockite, including:

pores with a size of 100 nm to 10 $\mu$m; and
pores with a size of 100 to 1000 $\mu$m.

A18.

A porous, bone graft material including whitlockite, including:

pores with a size of 100 nm to 10 $\mu$m; and
pores with a size of 100 to 1000 $\mu$m.

A19.

The bone graft material of any one of A17 and A18,
wherein the volume formed by pores with the size of 100 nm to 10 $\mu$m per unit mass of the bone graft material is 0.35 to 0.55 mL/g.

A20.

The bone graft material of any one of A17 and A18,

wherein the volume formed by pores with the size of 100 nm to 10 $\mu$m per unit mass of the bone graft material is 0.35 to 0.55 mL/g, and
the volume formed by pores with the size of 100 to 1000 $\mu$m per unit mass of the bone graft material is 0.7 to 1.6 mL/g.

A21.

A porous bone graft material consisting of whitlockite, including:

pores with a size of 100 nm to 100 $\mu$m; and
pores with a size of 100 to 1000 $\mu$m.

A22.

A porous bone graft material including whitlockite, including:

pores with a size of 100 nm to 100 $\mu$m; and
pores with a size of 100 to 1000 $\mu$m.

A23.

The bone graft material of any one of A21 and A22,
wherein the volume formed by pores with the size of 100 nm to 100 $\mu$m per unit mass of the bone graft material is 0.4 to 0.6 mL/g.

A24.

The bone graft material of any one of A21 and A22,

wherein the volume formed by pores with the size of 100 nm to 100 $\mu$m per unit mass of the bone graft material is 0.4 to 0.6 mL/g, and
the volume formed by pores with the size of 100 to 1000 $\mu$m per unit mass of the bone graft material is 0.7 to 1.6 mL/g.

A25.

The bone graft material of any one of A01 to A24, wherein the bone graft material has a porosity of 60% to 95%.

A26.

The bone graft material of any one of A01 to A25, wherein the bone graft material has a granular form.

A27.

The bone graft material of any one of A01 to A26, wherein the size of the bone graft material is 0.6 to 6 mm.

A28.

The bone graft material of any one of A01 to A26, wherein the size of the bone graft material is 0.6 to 1 mm.

A29.

The bone graft material of any one of A01 to A26, wherein the size of the bone graft material is 1 to 3 mm.

A30.

The bone graft material of any one of A01 to A26, wherein the size of the bone graft material is 3 to 6 mm.

A31.

The bone graft material of any one of A01 to A30, wherein the bone graft material has a compressive strength of 600 to 800 kPa.

A32.

The bone graft material of any one of A01 to A31,

wherein the bone graft material has a specific surface area of 5 to 6 $m^2$/g, and
wherein the specific surface area is analyzed by a Brunauer-Emmett-Teller method.

A33.

The bone graft material of any one of A01 to A32, wherein the bone graft material has a property of absorbing approximately 2.0 g or more of water per unit mass.

[Examples]

The invention provided by the specification will be described in further detail through experimental examples or examples. These experimental examples are merely provided to exemplify the content disclosed in the present application, and it will be apparent to those of ordinary skill in the art that the scope of the content disclosed by the specification is not to be construed as limited by these experimental examples.

**Experimental Examples**

**Preparation Example: Preparation of whitlockite bone graft material including various sizes of pores (Sample 1-1, Sample 1-2, Sample 1-3, and Sample 1-4)**

A primary slurry was prepared by mixing a vehicle, a binder, and a dispersant. Here, a silace-based compound was used as a vehicle, a PVA solution was used as a binder, and ammonium carboxylate was used as a dispersant. A secondary slurry was prepared by sieving whitlockite powder synthesized with a crystal size of 50 nm or less, and mixing it with the primary slurry in a mass ratio of 1:1.2. Afterward, using a 3-roll-mill process in which the secondary slurry and a polyurethane sponge were passed through three rollers, primary coating was performed on the surface and inside of the sponge, and then drying was performed. A primary sintered body was then prepared by performing primary thermal treatment at 750 °C for approximately 2 hours. Subsequently, a tertiary slurry was prepared by mixing whitlockite powder, a binder, and a dispersant in a mass ratio of 1:9:0.1, secondary coating was performed by dipping the primary sintered body in the tertiary slurry, and a secondary sintered body (Sample 1) was prepared by performing second thermal treatment at 750 °C for approximately 2 hours. Subsequently, whitlockite bone graft material granules (Sample 1-1) with a size of approximately 1.0 to 2.0 mm, whitlockite bone graft material granules (Sample 1-2) with a size of approximately 0.6 to 1.0 mm, whitlockite bone graft material granules (Sample 1-3) with a size of approximately 1 to 3 mm, and whitlockite bone graft material granules (Sample 1-4) with a size of approximately 3 to 6 mm were prepared, respectively, by grinding Sample 1 through a grinding process.

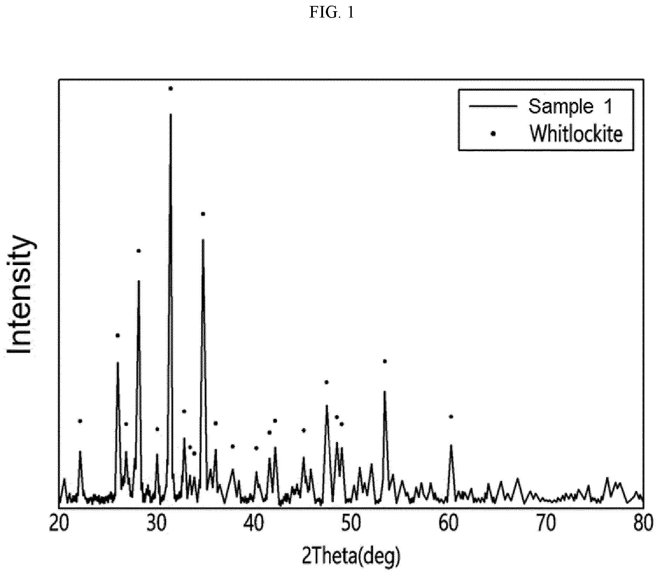

FIG. 1 shows the XRD data of a whitlockite bone graft material of the present application prepared according to Preparation Example. XRD analysis was performed after grinding Sample 1 into a fine powder. Sample 1-1, Sample 1-2, Sample 1-3, and Sample 1-4 were prepared by grinding Sample 1, and have the same composition (100% whitlockite).

The phase compositions of the bone graft materials prepared according to Preparation Example and Comparative Examples to be described below were determined using an X-ray diffractometer (XRD; Smart Lab, Rigaku, Germany). The XRD pattern of each bone graft material was recorded under the following measurement conditions using Cu K$\alpha$1 radiation at room temperature.

- Measurement conditions

Tube voltage: 40 kV
Tube current: 40 mA
Step size: 0.02, and
3°/min scan rate in angular range of 20°~ 80°

## Comparative Examples

*(1) Preparation and confirmation of Comparative Example 1*

Comparative Example 1: A 100% hydroxyapatite (HAp)-based alloplastic bone graft material (1.0 to 2.0 mm, 100% HAp, BONGROS Inc.) was prepared.

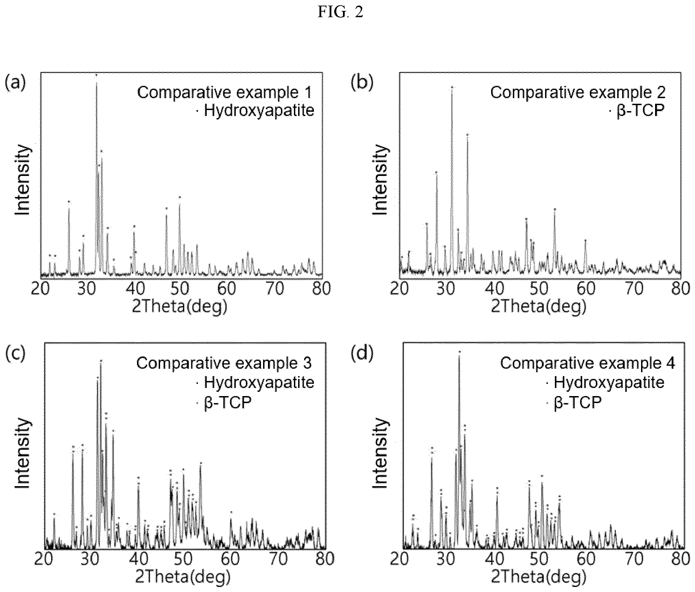

FIG. 2 shows the XRD data of bone graft materials of Comparative Examples. Specifically, in FIG. 2, (a) shows the XRD data of Comparative Example 1. In FIG. 2, (b) shows the XRD data of Comparative Example 2. In FIG. 2, (c) shows the XRD data of Comparative Example 3. In FIG. 2, (d) shows the XRD data of Comparative Example 4.

Referring to FIG. 2, it can be confirmed that the crystal structure of the bone graft material of Comparative Example 1 showed the pattern of the hydroxyapatite material. A secondary phase was not found.

*(2) Preparation and confirmation of Comparative Example 2*

Comparative Example 2: A 100% $\beta$-TCP-based alloplastic bone graft material (1.0 to 2.0 mm, 100% $\beta$-TCP, EXCELOS Inc.) was prepared.

Referring to FIG. 2 again, it can be confirmed that the crystal structure of the bone graft material of Comparative Example 2 showed the pattern of the $\beta$-TCP material. A secondary phase was not found.

*(3) Preparation and confirmation of Comparative Example 3*

Comparative Example 3: A heterologous bone graft material (0.6 to 1.0 mm, 30% HAp, 70% $\beta$-TCP, Boncel-Os Inc.) consisting of 30% hydroxyapatite and 70% $\beta$-TCP was prepared.

Referring to FIG. 2 again, it can be confirmed that the crystal structure of the bone graft material of Comparative Example 3 showed approximately 30% hydroxyapatite and approximately 70% $\beta$ tricalcium phosphate. A secondary phase was not found.

*(4) Preparation and confirmation of Comparative Example 4*

Comparative Example 4: A heterologous bone graft material (0.6 to 1.0mm, 60% HAp, 40% $\beta$-TCP, OSTEON III Inc.) consisting of 60% hydroxyapatite and 40% $\beta$-TCP was prepared.

Referring to FIG. 2 again, it can be confirmed that the crystal structure of the bone graft material of Comparative Example 4 showed approximately 60% hydroxyapatite and approximately 40% $\beta$-TCP. A secondary phase was not found.

*(5) Preparation of Comparative Example 5*

Comparative Example 5: A whitlockite bone graft material (3 to 6 mm-sized granules) including only nanopores was prepared by the following method.

Whitlockite powders synthesized with a crystal size of 50nm or less were sieved less to 150 $\mu$m or less. The result powders were added into a mold, and a uniaxial pressing was performed to prepare a molded body. Afterward, a sintered body was prepared by performing thermal treatment at 750 °C for approximately 2 hours. Subsequently, bone graft material granules with a size of approximately 3.0 to 6.0 mm were prepared through a grinding process.

*(6) Preparation of Comparative Example 6*

Comparative Example 6: A whitlockite bone graft material (3 to 6 mm-sized granules) including only macropores was prepared by the following method.

Whitlockite powders synthesized with a crystal size of 200nm or less were sieved less to 150 $\mu$m or less. The sieved powders, DI water, and dispersant (ammonium carboxylate) were mixed in a mass ratio of 10:10:0.6 to prepare a slurry. The prepared slurry was cast in a mold of plaster and polypropylene and dried in a 60 °C dry oven for 24 hours. Subsequently, after a sintered body was prepared by removing the mold and performing thermal treatment at 1000 °C for approximately 2 hours, bone graft material granules with a size of approximately 3.0 to 6.0 mm were prepared through a grinding process.

**SEM analysis**

*SEM analysis*

The morphologies of the bone graft materials prepared according to Preparation Example and Comparative Examples were analyzed through SEM. Specifically, a XL-30-FEG scanning electron microscope (SEM, Philips) was used under an accelerating voltage of 15 kV.

*Bone graft material prepared according to Preparation Example*

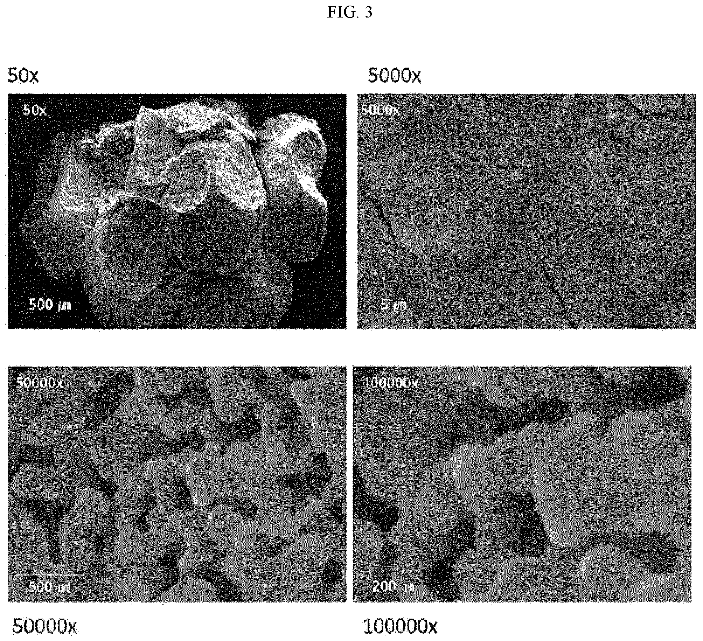

FIG. 3 shows the SEM data of the bone graft material (Sample 1-3) prepared according to Preparation Example. Specifically, FIG. 3 shows the SEM data of the bone graft material prepared according to Preparation Example, taken at magnifications of 50-fold (50x), 5000-fold (5000x), 50000-fold (50000x), and 100000-fold (100000x).

Referring to FIG. 3, it can be seen that the bone graft material according to an embodiment of the present application, prepared according to Preparation Example, included all of macropores (pores with a diameter of 100 $\mu$m or more), micropores (pores with a diameter of 1 to 100 $\mu$m), and nanopores (pores with a diameter of 1000 nm or less).

*Comparative Examples 1 to 4*

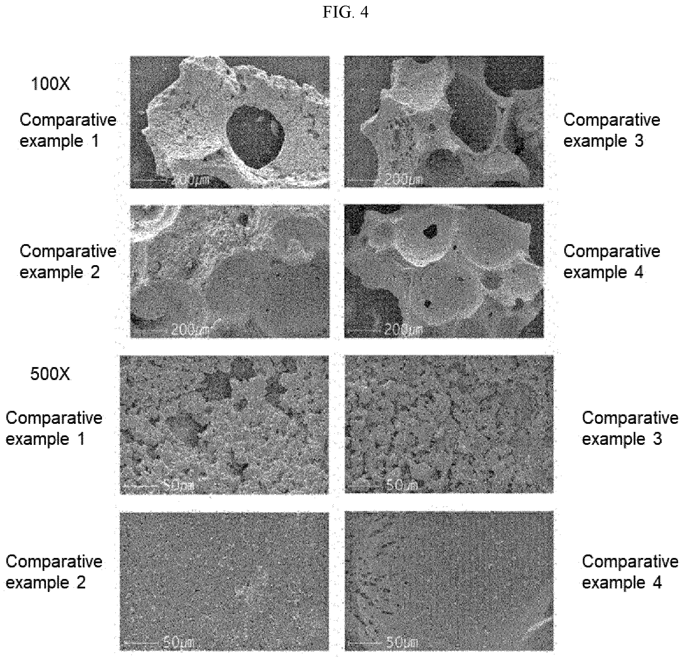

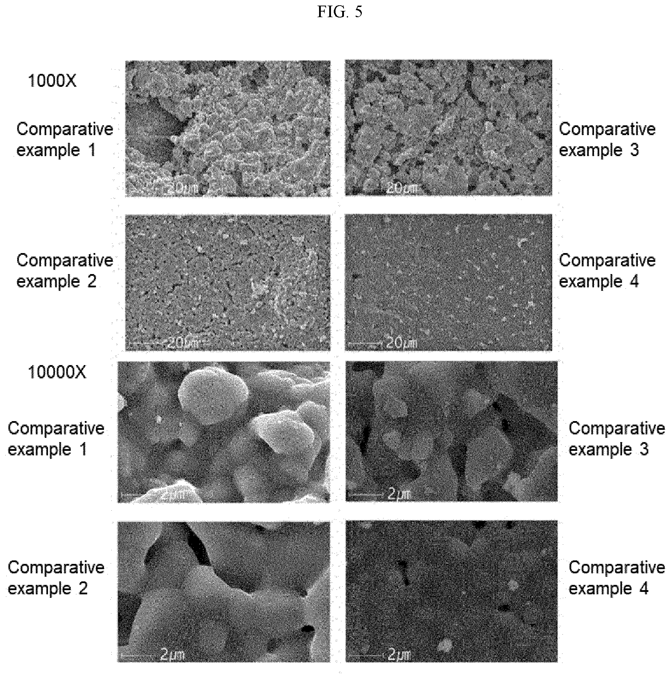

FIGS. 4 and 5 show the SEM data of the bone graft materials of Comparative Examples 1 to 4. It can be visually confirmed that, in the bone graft materials of Comparative Examples 1 to 4, pores of 10 $\mu$m or more were observed, but pores with a size of less than 1000 nm were not observed.

*Comparative Examples 5 and 6*

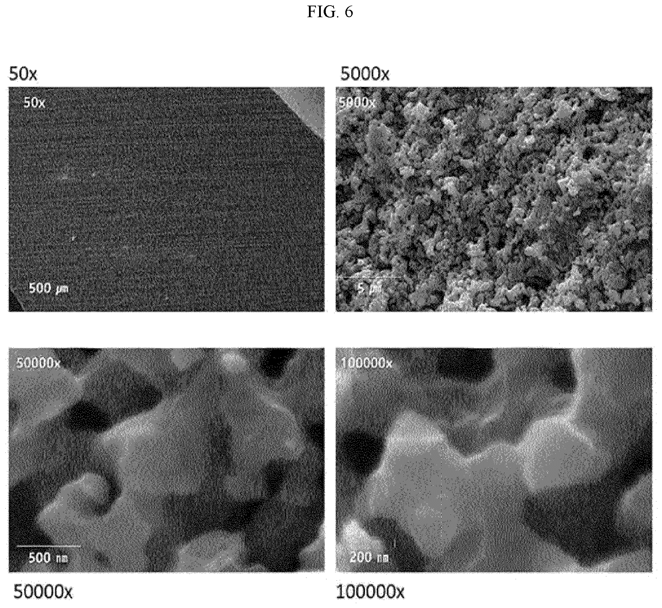

FIG. 6 shows the SEM data of the bone graft material of Comparative Example 5. It can be visually confirmed that the bone graft material of Comparative Example 5 included only nanopores.

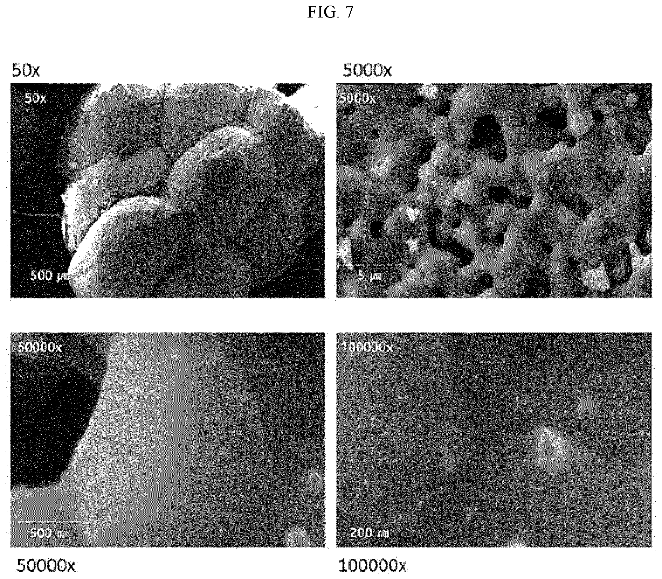

FIG. 7 shows the SEM data of the bone graft material of Comparative Example 6. It can be visually confirmed that only micropores and macropores were observed in the bone graft material of Comparative Example 6.

**Analysis of porosity and pore distribution**

*Analysis method for porosity and pore distribution*

Porosities and pore sizes of the bone graft materials prepared according to Preparation Examples and Comparative Examples were analyzed, respectively. The porosity and pore size of each bone graft material were analyzed under a pressure range of 0.20 to 33000 psia using a mercury porosimeter (MicroActive AutoPore V 9600, Micromeritics Inst Inc., USA).

*Bone graft materials prepared according to Preparation Examples*

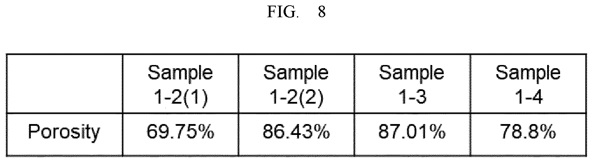

FIG. 8 shows the results of analyzing porosities of the bone graft materials prepared according to Preparation Example. The porosity of the bone graft material of Sample 1-2 ranged from 58.6% to 80.9%, and its median value was observed to be 69.75% (primary measurement). According to another measurement, the porosity of the bone graft material of Sample 1-2 was observed to be 86.43% (secondary measurement). The porosity of the bone graft material of Sample 1-3 was observed to be 87.01%. The porosity of the bone graft material of Sample 1-4 was observed to be 78.8%.

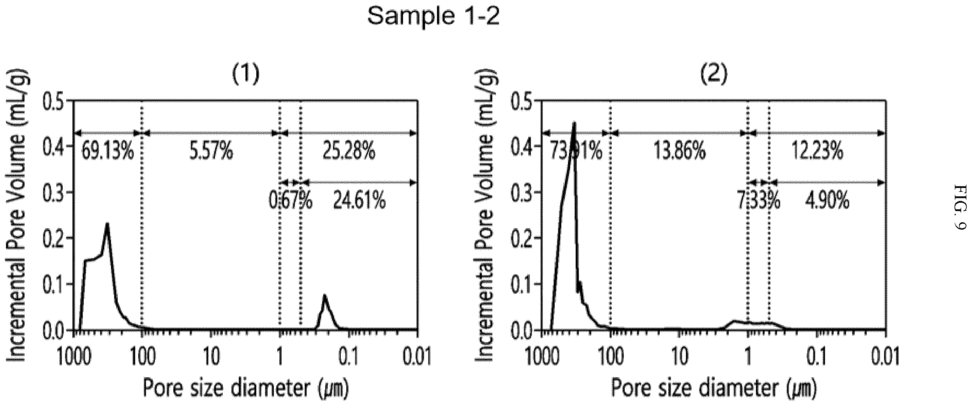

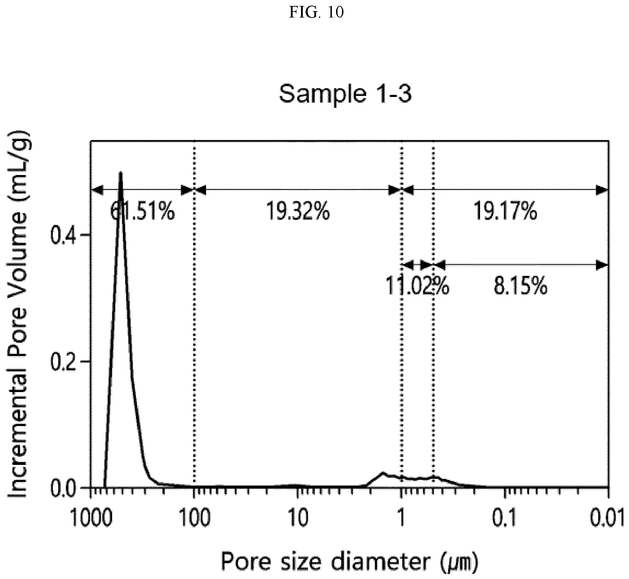

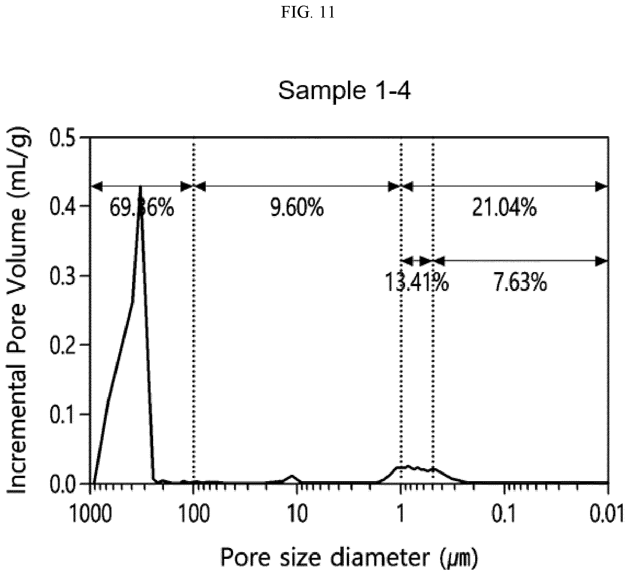

FIGS. 9 to 11 are graphs showing the results of analyzing the pore distribution of the bone graft materials prepared according to Preparation Examples. Specifically, FIG. 9 is a graph showing the results of analyzing the pore distribution of bone graft material of Sample 1-2 (size: 0.6-1 mm). In FIG. 9, Sample 1-2(1) and Sample 1-2(2) show results for different measurements. FIG. 10 is a graph showing the results of analyzing the pore distribution of the bone graft material of Sample 1-3 (size: 1-3 mm). FIG. 11 is a graph showing the results of analyzing the pore distribution of the bone graft material of Sample 1-4 (size: 3-6 mm). In each sample, the vol% of pores by pore size was analyzed (in FIGS. 9 to 11, values expressed as %). Here, the "vol% of pores" refers to the '(the volume formed by pores of a corresponding size in a unit mass)/(the total volume formed by all pores in a unit mass)'. Specifically, the vol% of nanopores, and the vol% of micropores were measured.

-   vol% of nanopores: volume occupied by pores with diameter of 1000 nm or less in unit mass / total volume occupied by all pores in unit mass

- vol% of micropores: volume occupied by pores with diameter of 1 to 100 $\mu$m in unit mass / total volume occupied by all pores in unit mass
- vol% of macropores: volume occupied by pores with diameter of 100 $\mu$m or more in unit mass / total volume occupied by all pores in unit mass

The volume and vol% by measured pore size for each sample are shown in Tables 1 to 4 below. Tables 1 to 4 below specify the data disclosed in FIGS. 9 to 11 by pore size section. Specifically, Table 1 shows the results of analyzing the pore size distribution of Sample 1-2(1). Table 2 shows the results of analyzing the pore size distribution of Sample 1-2(2). Table 3 shows the results of analyzing the pore size distribution of Sample 1-3. Table 4 shows the results of analyzing the pore size distribution of Sample 1-4. In the following tables, the volume (mL/g) refers to a volume formed by pores of a corresponding size per unit mass of a bone graft material. The vol% was calculated by "100 x (volume formed by pores of corresponding size in unit mass)/(total volume formed by all pores in unit mass)".

Table 1. Results of analyzing pore size distribution of Sample 1-2 (1)

| Sample 1-2 (1) | | |
|---|---|---|
| Size of pores ($\mu$m) | Volume (mL/g) | Vol% |
| -0.1 | 0.004 | 0.23 |
| 0.1-0.5 | 0.37 | 24.38 |
| 0.5-1 | 0.01 | 0.67 |
| 1-10 | 0.02 | 1.39 |
| 10-100 | 0.06 | 4.18 |
| 100-1000 | 1.02 | 69.13 |
| Total | 1.48 | |
| Values calculated from other ranges | | |
| Size of pores ($\mu$m) | Volume (mL/g) | Vol% |
| 0.1-1 | 0.38 | 25.05 |
| 0.1-10 | 0.4 | 26.44 |
| 0.1-100 | 0.46 | 30.62 |
| 1-100 | 0.08 | 5.57 |

Table 2. Results of analyzing pore size distribution of Sample 1-2 (2)

| Sample 1-2 (2) | | |
|---|---|---|
| Size of pores ($\mu$m) | Volume (mL/g) | Vol% |
| 0.1-0.5 | 0.10 | 4.89 |
| 0.5-1 | 0.15 | 7.33 |
| 1-10 | 0.21 | 10.50 |
| 10-100 | 0.07 | 3.36 |
| 100-1000 | 1.50 | 73.91 |
| Total | 2.03 | |
| Values calculated from other ranges | | |
| Size of pores ($\mu$m) | Volume (mL/g) | Vol% |
| 0.1-1 | 0.25 | 12.22 |
| 0.1-10 | 0.46 | 22.72 |

(continued)

| Values calculated from other ranges | | |
|---|---|---|
| Size of pores ($\mu$m) | Volume (mL/g) | Vol% |
| 0.1-100 | 0.53 | 26.08 |
| 1-100 | 0.28 | 13.86 |

Table 3. Results of analyzing pore size distribution of Sample 1-3

| Sample 1-3 | | |
|---|---|---|
| Size of pores ($\mu$m) | Volume (mL/g) | Vol% |
| 0.1-0.5 | 0.11 | 8.14 |
| 0.5-1 | 0.15 | 11.02 |
| 1-10 | 0.22 | 15.95 |
| 10-100 | 0.05 | 3.37 |
| 100-1000 | 0.84 | 61.51 |
| total | 1.37 | |
| Values calculated from other ranges | | |
| Size of pores ($\mu$m) | Volume (mL/g) | Vol% |
| 0.1-1 | 0.26 | 19.16 |
| 0.1-10 | 0.48 | 35.11 |
| 0.1-100 | 0.53 | 38.48 |
| 1-100 | 0.27 | 19.32 |

Table 4. Results of analyzing pore size distribution of Sample 1-4

| Sample 1-4 | | |
|---|---|---|
| Size of pores ($\mu$m) | Volume (mL/g) | Vol% |
| 0.1-0.5 | 0.13 | 7.63 |
| 0.5-1 | 0.22 | 13.41 |
| 1-10 | 0.11 | 6.78 |
| 10-100 | 0.05 | 2.82 |
| 100-1000 | 1.14 | 69.36 |
| total | 1.65 | |
| Values calculated from other ranges | | |
| Size of pores ($\mu$m) | Volume (mL/g) | Vol% |
| 0.1-1 | 0.35 | 21.04 |
| 0.1-10 | 0.46 | 27.82 |
| 0.1-100 | 0.50 | 30.64 |
| 1-100 | 0.16 | 9.60 |

*Bone graft materials prepared according to Comparative Examples*

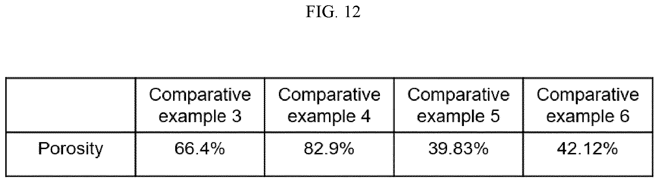

FIG. 12 shows the results of analyzing the bone graft materials of Comparative Examples.

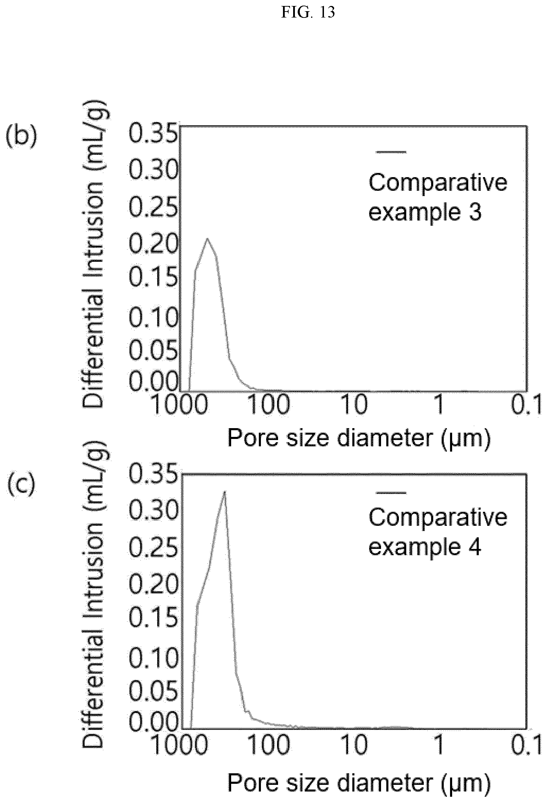

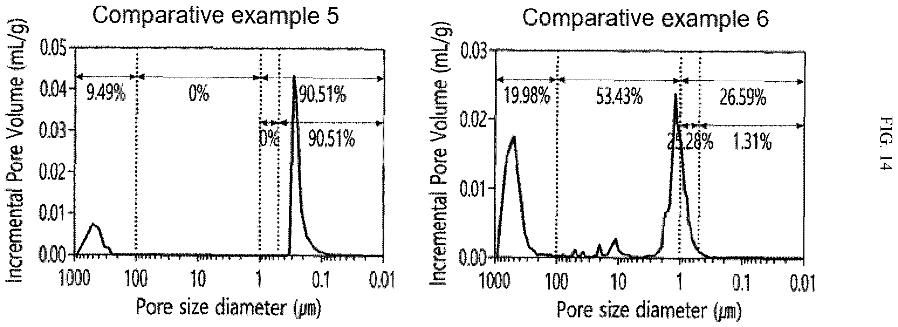

FIGS. 13 and 14 show the results of analyzing the pore distribution of the bone graft materials prepared according to Comparative Examples. Specifically, FIG. 13 shows the results of analyzing the pore distribution of bone graft materials prepared according to Comparative Examples 3 and 4. FIG. 14 shows the results of analyzing the pore distribution of bone graft materials prepared according to Comparative Examples 5 and 6.

Table 5 below specifies the data for Comparative Example 5 and Comparative Example 6 disclosed in FIG. 14 by pore size section.

Table 5. Results of analyzing pore size distribution of Comparative Example 5 and Comparative Example 6

| Comparative example 5 | | |
| --- | --- | --- |
| Size of pores ($\mu$m) | Volume (mL/g) | Vol% |
| 0.1-0.5 | 0.20 | 88.76 |
| 0.5-1 | 0.00 | 0.00 |
| 1-10 | 0.00 | 0.00 |
| 10-100 | 0.00 | 0.00 |
| 100-1000 | 0.02 | 9.49 |
| total | 0.23 | |
| Comparative example 6 | | |
| Size of pores ($\mu$m) | Volume (mL/g) | Vol% |
| 0.1-0.5 | 0.003 | 1.31 |
| 0.5-1 | 0.07 | 25.28 |
| 1-10 | 0.13 | 47.65 |
| 10-100 | 0.02 | 5.77 |
| 100-1000 | 0.05 | 19.98 |
| total | 0.27 | |

**Specific surface area analysis**

*Method of analyzing specific surface area*

Specific surface areas of the bone graft materials prepared according to Preparation Example and Comparative Examples were analyzed. The specific surface area of each bone graft material was evaluated by a Brunauer-Emmett-Teller (BET, Tristar II 3020, Micromeritics Inst Inc., USA) device utilizing an N2 adsorption method for a pore size distribution of 2 to 300 nm.

*Results of analyzing specific surface area*

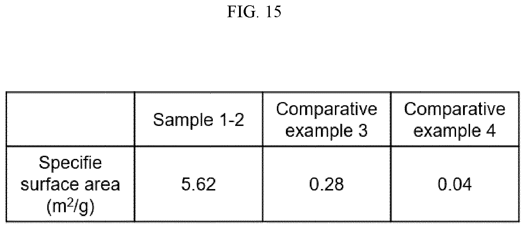

FIG. 15 shows the results of measuring the specific surface areas of the bone graft materials prepared according to Preparation Example (Sample 1-2) and Comparative Examples.

The specific surface area of the bone graft material prepared according to Preparation Example was evaluated to be approximately 5.62 $m^2$/g, the specific surface area of the bone graft material prepared according to Comparative Example 3 was evaluated to be approximately 0.28 $m^2$/g, and the specific surface area of the bone graft material prepared according to Comparative Example 4 was evaluated to be approximately 0.04 $m^2$/g.

According to the above analysis results, it was confirmed that the specific surface area of the bone graft material prepared according to Preparation Example is considerably larger than those of the bone graft materials prepared according to Comparative Examples 3 and 4.

Considering this, as the bone graft material according to an embodiment of the present application has a relatively high specific surface area, the area that is in contact with blood and cells in the body can be expanded. In addition, based on this characteristic, the bone graft material according to one embodiment of the present application may have enhanced osteogenic ability compared to Comparative Examples 3 and 4.

*Measurement of specific surface areas of bone graft materials of Sample 1-3, Sample 1-4, Comparative Example 5 and Comparative Example 6*

The specific surface area of the bone graft material of Sample 1-3 is measured in accordance with the content in "Method of analyzing specific surface area" in Experimental Example of the present application.

The specific surface area of the bone graft material of Sample 1-4 is measured in accordance with the content in "Method of analyzing specific surface area" in Experimental Example of the present application.

The specific surface area of the bone graft material of Comparative Example 5 is measured in accordance with the content in "Method of analyzing specific surface area" in Experimental Example of the present application.

The specific surface area of the bone graft material of Comparative Example 6 is measured in accordance with the content in "Method of analyzing specific surface area" in Experimental Example of the present application.

**Wettability analysis**

*Method of analyzing wettability*

The wettability of each of the bone graft materials prepared according to Preparation Examples and Comparative Examples was analyzed. The wettability of each bone graft material was evaluated by measuring the mass of water absorbed per g of the bone graft material over time using an Attention Sigma 700 model (Biolin Scientific).

*Results of analyzing wettability*

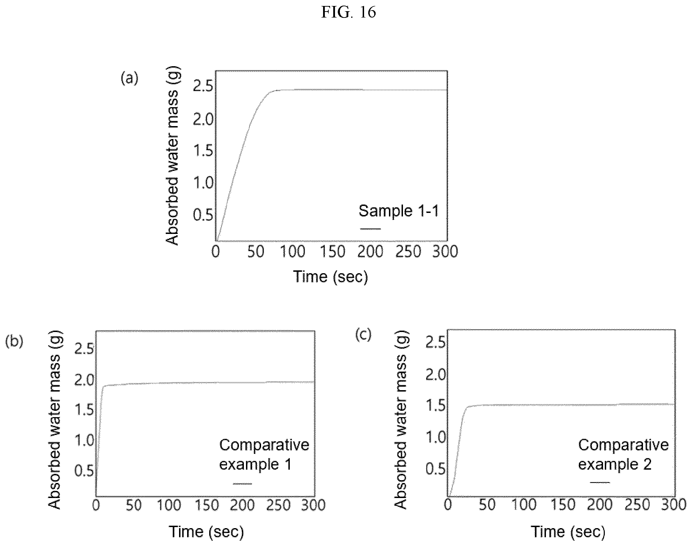

FIG. 16 shows the results of measuring the wettability of the bone graft materials prepared according to Preparation Example and Comparative Examples. Specifically, in FIG. 16, (a) is a graph showing the results of analyzing the wettability of the bone graft material (Sample 1-1) prepared according to Preparation Example. In FIG. 16, (b) is a graph showing the results of analyzing the wettability of the bone graft material prepared according to Comparative Example 1. In FIG. 16, (c) is a graph showing the results of analyzing the wettability of the bone graft material prepared according to Comparative Example 2.

Referring to FIG. 16, it can be confirmed that the bone graft material prepared according to Preparation Example has a property of absorbing up to approximately 2.5 g of water per unit mass. One the other hand, the bone graft material of Comparative Example 1 has a property of absorbing up to approximately 1.93 g of water per unit mass, and the bone graft material of Comparative Example 2 has a property of absorbing up to approximately 1.56 g of water per unit mass. That is, it was confirmed that the bone graft material prepared according to Preparation Example has a higher maximum mass of water that can be absorbed per unit mass than the bone graft materials according to Comparative Examples.

*Wettability of bone graft materials of Sample 1-3, Sample 1-4, Comparative Example 5 and Comparative Example 6*

The wettability of the bone graft material of Sample 1-2 is measured in accordance with the content in "Method of analyzing wettability" in Experimental Example of the present application.

The wettability of the bone graft material of Sample 1-3 is measured in accordance with the content in "Method of analyzing wettability" in Experimental Example of the present application.

The wettability of the bone graft material of Sample 1-4 is measured in accordance with the content in "Method of analyzing wettability" in Experimental Example of the present application.

The wettability of the bone graft material of Comparative Example 5 is measured in accordance with the content in "Method of analyzing wettability" in Experimental Example of the present application.

The wettability of the bone graft material of Comparative Example 6 is measured in accordance with the content in "Method of analyzing wettability" in Experimental Example of the present application.

**Compressive strength analysis**

*Method of analyzing compressive strength*

The compressive strength of each of the bone graft materials prepared according to Preparation Example and Comparative Examples was analyzed. Each bone graft material was prepared as a cuboid-shaped specimen with a size of 40*60*5 mm, and after setting a loading speed to 0.5 mm/min and the limit of distance to 2.5 mm, the compressive stress immediately before the bone graft material specimen was destroyed was measured while gradually increasing the compressive stress on each bone graft material. The compressive strength was measured before grinding into particles. For example, the compressive strength for Sample 1 was measured.

*Analysis results*

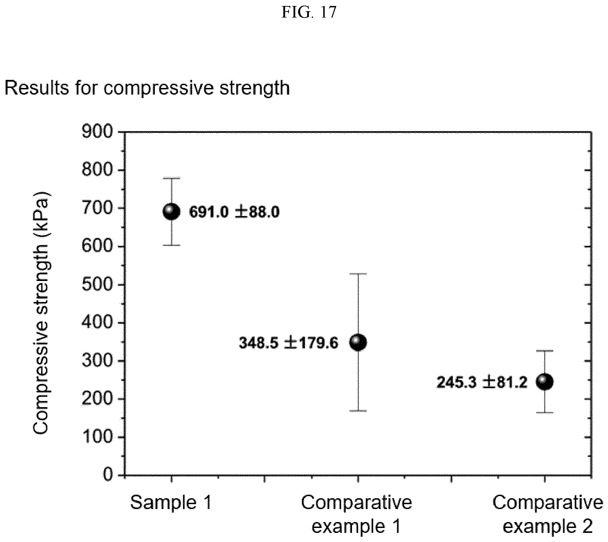

FIG. 17 shows the compressive strength of the bone graft materials prepared according to Preparation Example and Comparative Examples. Specifically, FIG. 17 shows the results of analyzing the compressive strength of bone graft materials prepared according to the bone graft material (Sample 1) prepared according to Preparation Example and bone graft materials prepared according to Comparative Examples.

Referring to FIG. 17, the bone graft material prepared according to Preparation Example maintained mechanical strength up to a compressive stress ranging from 603 to 780 KPa. On the other hand, the bone graft material of Comparative Example 1 maintained mechanical strength up to a compressive stress ranging from approximately 168.9 to 528.1 KPa. In addition, the bone graft material of Comparative Example 2 maintained mechanical strength up to a compressive stress ranging from approximately 164.1 to 326.5 KPa. In other words, it was confirmed that the bone graft material prepared according to Preparation Example has a higher compressive strength than the bone graft materials according to Comparative Examples.

According to the above analysis results, it can be confirmed that the bone graft material prepared according to Preparation Example has a relatively higher compressive strength than the bone graft materials prepared according to Comparative Examples 1 and 2. Accordingly, it can be inferred that the bone graft material according to an embodiment of the present application has relatively higher mechanical strength. Therefore, the bone graft material according to an embodiment of the present application may be more advantageous in securing a space where new bone will be formed during the period of new bone formation. In addition, the bone graft material according to an embodiment of the present application may prevent the destruction of particles constituting the bone graft material, and thus the manipulation of the bone graft material may be relatively easier during implantation.

*Compressive strength of bone graft materials of Comparative Examples 5 and 6*

The compressive strength of the bone graft material of Comparative Example 5 is measured in accordance with the content in "Method of analyzing compressive strength" in Experimental Example of the present application.

The compressive strength of the bone graft material of Comparative Example 6 is measured in accordance with the content in "Method of analyzing compressive strength" in Experimental Example of the present application.

**Thermogravimetric analysis**

*Analysis method*

The thermal strength of each bone graft material was evaluated by measuring the total weight loss of the bone graft materials prepared according to Preparation Example and Comparative Examples using thermogravimetric analysis. Specifically, using a thermogravimetry/differential thermal analyzer (TG-DTA; STA 409 PC, NETZSCH, Germany), the changing weight of the sample was measured while gradually heating each bone graft material specimen at a heating rate of 10 °C/min from room temperature to approximately 1300 °C.

*Analysis results*

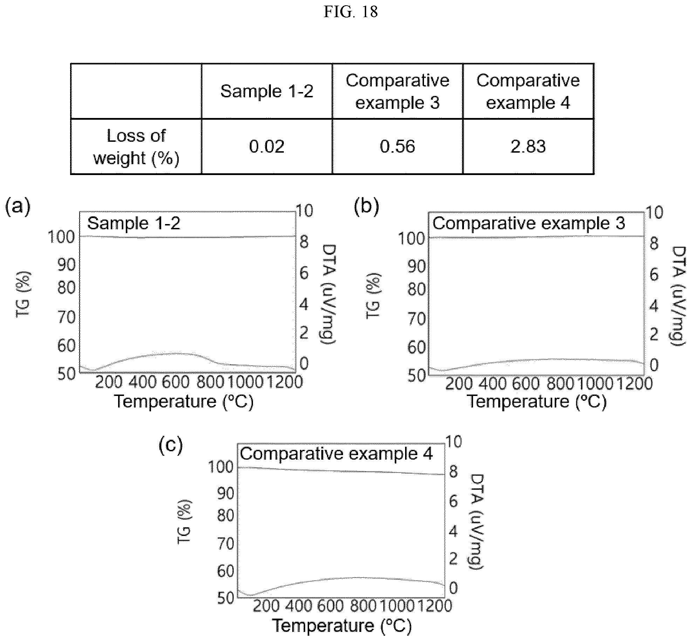

FIG. 18 shows the results of thermogravimetric analysis of the bone graft materials prepared according to Preparation Example and Comparative Examples. Specifically, FIG. 18 shows the table and graph showing the results of thermogravimetric analysis for the bone graft material (Sample 1-2) prepared according to Preparation Example and the bone graft materials prepared according to Comparative Examples. In FIG. 18, (a) is a graph showing the results of thermogravimetric analysis for the bone graft material prepared according to Preparation Example. In FIG. 18, (b) is a graph showing the results of thermogravimetric analysis for the bone graft material prepared according to Comparative Example 3. In FIG. 18, (c) is a graph showing the results of thermogravimetric analysis for the bone graft material prepared according to Comparative Example 4.

Referring to FIG. 18, the bone graft material prepared according to Preparation Example lost only 0.02% of its total mass while increasing a heating temperature until 1300 °C. On the other hand, the bone graft material prepared according to Comparative Example 3 lost 0.56% of its total mass while increasing a heating temperature until 1300 °C, and the bone graft material prepared according to Comparative Example 4 lost 2.83% of its total mass under the same conditions. In other words, the bone graft material prepared according to Preparation Example has a higher resistance to heat than the bone graft materials according to Comparative Examples.

From the above analysis results, it was confirmed that the bone graft material according to an embodiment of the present application also has higher mechanical strength and higher thermal strength than the bone graft materials prepared according to Comparative Examples 3 and 4.

*Thermogravimetry of bone graft materials of Comparative Example 5 and Comparative Example 6*

The thermogravimetry of the bone graft material of Comparative Example 5 is measured in accordance with the content in "Thermogravimetric analysis method" in Experimental Example of the present application.

The thermogravimetry of the bone graft material of Comparative Example 6 is measured in accordance with the content in "Thermogravimetric analysis method" in Experimental Example of the present application.

## Ion release

*Method of analyzing ion release*

The ion release patterns of the bone graft materials prepared according to Preparation Example and Comparative Examples were evaluated, respectively. Specifically, 1.0 g of each bone graft material was precipitated in 5 mL of DI water for 30 minutes, 1, 2, 3, 7, 14, or 21 days at room temperature, and 0.5 mL aliquots were eluted at each time, followed by exchange with a new solution. In addition, the eluate eluted at each time was filtered using a syringe, and the amount of elements present in the eluate was evaluated as the amount of elements released over precipitation time using inductively-coupled plasma atomic emission spectrometry (ICP-AES).

*Analysis results*

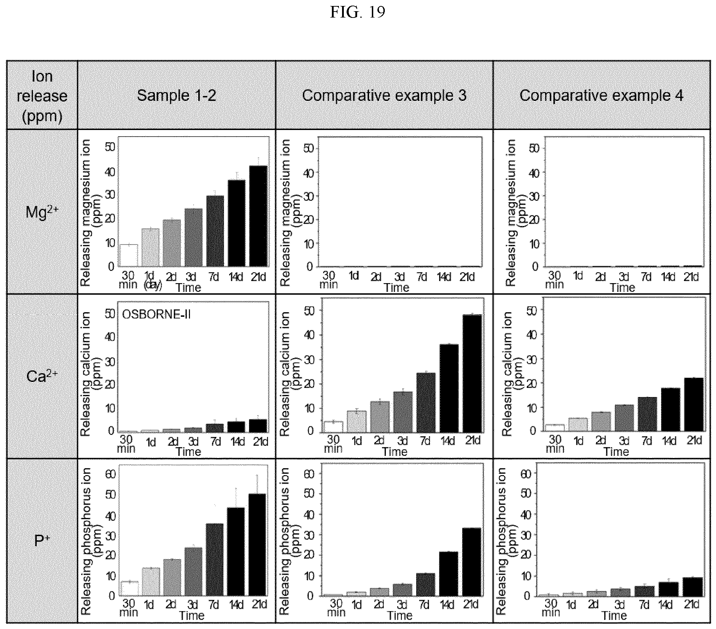

FIG. 19 shows the results of analyzing the ion release of the bone graft materials prepared according to Preparation Example and Comparative Examples. Specifically, FIG. 19 shows the types of released ions and the results of analyzing ion release amounts of the bone graft material (Sample 1-2) prepared according to Preparation Example and the bone graft materials prepared according to Comparative Examples.

Referring to FIG. 19, it can be confirmed that the bone graft material prepared according to Preparation Example releases magnesium ions, calcium ions, and phosphorus ions. On the other hand, it was confirmed that the bone graft materials prepared according to Comparative Examples 3 and 4 do not release magnesium ions but release calcium ions and phosphorus ions.

Particularly, the bone graft material prepared according to Preparation Example was analyzed as releasing magnesium ions at a high concentration level of approximately 45 ppm.

As described above, it is known that magnesium ions serve to promote osteogenesis *in vivo.* The bone graft material prepared according to Preparation Example may release magnesium ions from its surface into the body, and therefore, the bone graft material according to an embodiment of the present application may exhibit a more advantageous effect on new bone formation at an implantation site.

*Ion release of bone graft materials of Sample 1-3, Sample 1-4, Comparative Example 5 and Comparative Example 6*

The degree of ion release of the bone graft material of Sample 1-3 is measured in accordance with the content in "Method of analyzing ion release" in Experimental Example of the present application.

The degree of ion release of the bone graft material of Sample 1-4 is measured in accordance with the content in "Method of analyzing ion release" in Experimental Example of the present application.

The degree of ion release of the bone graft material of Comparative Example 5 is measured in accordance with the content in "Method of analyzing ion release" in Experimental Example of the present application.

The degree of ion release of the bone graft material of Comparative Example 6 is measured in accordance with the content in "Method of analyzing ion release" in Experimental Example of the present application.

## Osteogenic ability

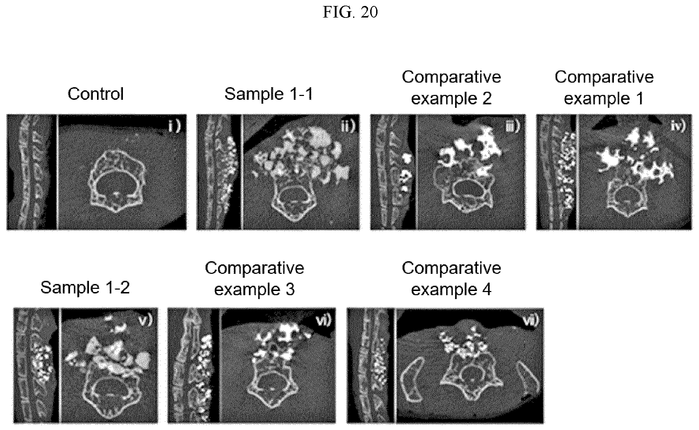

Hereinafter, referring to FIGS. 20 to 24, various analysis methods and analysis results for evaluating properties associated with the osteogenic ability of the bone graft materials prepared according to Preparation Examples and Comparative Examples will be described in detail.

Hereinafter, the analysis methods and analysis results used to evaluate osteogenic ability will be described in detail.

Ten 6-week-old C57BL/6 mice were prepared in each experimental group. Each of the bone graft materials prepared according to Preparation Examples and Comparative Examples was inserted between the two transverse processes of each mouse vertebra. For each experimental group, at 6 weeks after implantation of the bone graft material, the new bone volume (NV) and the total bone volume (TV) at the implantation site were evaluated using various analysis methods to be described below.

A group of mice that underwent spinal fusion surgery was used as a control, and a first group into which the whitlockite-based bone graft materials (Sample 1-1 and Sample 1-2) prepared according to Preparation Examples were inserted, a second group into which the hydroxyapatite-based bone graft material prepared according to Comparative Example 1 was inserted, a third group into which the β-TCP-based bone graft material prepared according to Comparative Example 2 was inserted, a fourth group into which the bone graft material consisting of 30% hydroxyapatite and 70% β-TCP, prepared according to Comparative Example 3, was inserted, and a fifth group into which the bone graft material consisting of 60% hydroxyapatite and 40% β-TCP, prepared according to Comparative Example 4 were used as "experimental groups" to evaluate osteogenic ability.

Here, the new bone volume (NV) is a measurement value of the amount of newly generated bone, and is meaningful in that it indicates the extent to which a bone graft material induces bone regeneration. In addition, the total bone volume (TV) is the sum of NV and an amount of the remaining bone graft material, and is meaningful in that the bone graft material remains and provides a framework.

Hereinafter, the analysis methods and analysis results used to evaluate osteogenic ability will be described in detail.

Micro-CT analysis was used as one of the analysis methods for evaluating osteogenic ability. Specifically, the skull of the control and the skulls of experimental groups containing the implanted bone graft material, which were prepared according to the above-described experimental protocols, were obtained, and a micro-CT image of the skull of each group was acquired with a resolution of 13.85 μm (achieved using 130kV and 60μA) using a high-resolution Skyscan1173 micro-CT system (Bruker-microCT, Kartuizersweg 3B 2550 Kontich, Belgium). A 1.0 mm-thick aluminum filter was used, a total exposure time for imaging was 500 ms. The collected micro-CT images were reconstructed with Nrecon software (Ver. 1.7.4.6, Bruker-microCT, Kartuizersweg 3B 2550 Kontich, Belgium).

Subsequently, the diameter of a region of interest (ROI) was set to 8 mm, and the reconstructed micro-CT images were analyzed with image analysis software (Image Pro Plus, Ver.7.0, Media Cybernetics Inc., Rockville, USA) to measure a new bone volume (NV), a remaining bone substitute volume (BV), and a total bone volume (the sum of NV and BV).

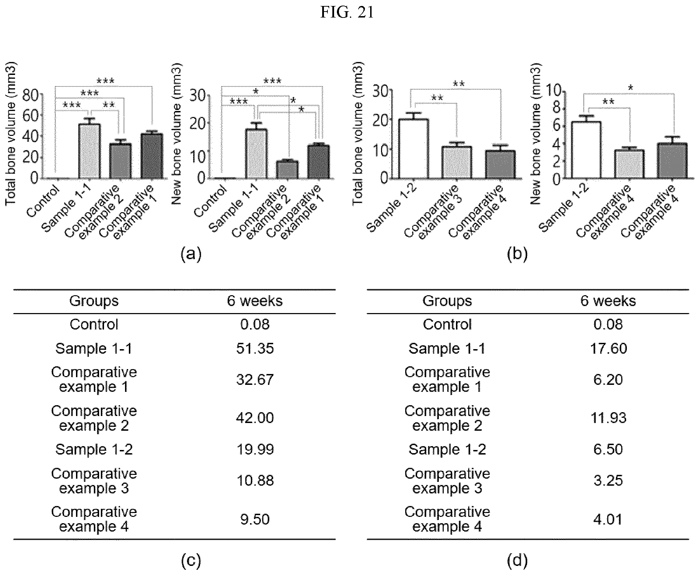

FIG. 20 is a set of micro-CT images acquired through micro-CT analysis. FIG. 21 shows graphs and tables illustrating the NV and TV of the control and experimental groups measured by analyzing micro-CT. Specifically, in FIGS. 21, (a) and (b) are graphs illustrating the NV and TV of the control and experimental groups. In FIG. 21, (c) shows the table illustrating the TV of the control and experimental groups. In FIG. 21, (d) shows the table illustrating the NV of the control and experimental groups.

Referring to FIGS. 20 and 21, it was confirmed that, in all experimental groups into which a bone graft material was inserted, the new bone volume (NV) and the total bone volume (TV) were measured to be significantly higher than those of the control into which no bone graft material was inserted. Specifically, at 6 weeks after the implantation of the bone graft material, the total bone volume (TV) was measured to be approximately 0.08 mm$^3$ for the control, the total bone volume (TV) was measured to be 51.35 mm$^3$ for the bone graft material of Sample 1-1, and the total bone volume (TV) was measured to be 19.99 mm$^3$ for the bone graft material of Sample 1-2. In addition, at 6 weeks after the implantation of the bone graft material, the new bone volume (NV) was measured to be approximately 0.08 mm$^3$ for the control, the new bone volume (NV) was measured to be 17.60 mm$^3$ for the bone graft material of Sample 1-1, and the new bone volume (NV) was measured to be 6.50 mm$^3$ for the bone graft material of Sample 1-2.

In addition, in the first group into which the whitlockite-based bone graft material of Sample 1-1 was inserted, it was proven that the new bone volume (NV) and the total bone volume (TV) were measured significantly higher than those of other groups into which an "same kind" bone graft material was inserted. Specifically, the total bone volume (TV) was measured to be 51.35 mm$^3$ for the bone graft material of Sample 1-1, the total bone volume (TV) was measured to be 32.67 mm$^3$ for the bone graft material prepared according to Comparative Example 1, and the total bone volume (TV) was measured to be 42.00 mm$^3$ for the bone graft material prepared according to Comparative Example 2. In addition, the new bone volume (NV) was measured to be 17.60 mm$^3$ for the bone graft material of Sample 1-1, the new bone volume (NV) was measured to be 6.20 mm$^3$ for the bone graft material prepared according to Comparative Example 1, and the new bone volume (NV) was measured to be 11.93 mm$^3$ for the bone graft material prepared according to Comparative Example 2.

In summary, in the total bone volume (TV) evaluation, the TV value of the first group (Sample 1-1) was approximately 1.5 times higher than that of the second group (Comparative Example 1) into which the hydroxyapatite-based bone graft material was inserted. In addition, it was confirmed that the TV value of the first group was approximately 1.2 times higher than that of the third group (Comparative Example 2) into which the β-TCP-based bone graft material was inserted.

In addition, in the new bone volume (NV) evaluation, the NV value of the first group (Sample 1-1) was approximately 2.8 times higher than that of the second group (Comparative Example 1) into which the hydroxyapatite-based bone graft material was inserted. In addition, the NV value of the first group was approximately 1.4 times higher than the TV value of the third group (Comparative Example 2) into which the β-TCP-based bone graft material was inserted.

In addition, in the case of the first group into which the whitlockite-based bone graft material prepared according to Preparation Example (the bone graft material of Sample 1-2) was inserted, it was proven that the new bone volume (NV) and the total bone volume (TV) were measured to be significantly higher than those of the group into which the β-TCP-HAp composite graft material was inserted. Specifically, the total bone volume (TV) was measured to be 19.99 mm$^3$ for the bone graft material of Sample 1-2, the total bone volume (TV) was measured to be 10.88 mm$^3$ for the bone graft material prepared according to Comparative Example 3, and the total bone volume (TV) was measured to be 9.5 mm$^3$ for the bone graft material prepared according to Comparative Example 4. In addition, the new bone volume (NV) was measured to be 6.5 mm$^3$ for the bone graft material of Sample 1-2, the new bone volume (NV) was measured to be 3.25 mm$^3$ for the bone graft material prepared according to Comparative Example 3, and the new bone volume (NV) was measured to be 4.01 mm$^3$ for the bone graft material prepared according to Comparative Example 4.

In the total bone volume (TV) evaluation, the TV value of the first group (Sample 1-2) was approximately 1.8 times higher than that of the fourth group (Comparative Example 3). In addition, it was confirmed that the TV value of the first group was approximately 2.1 times higher than that of the fifth group (Comparative Example 4).

Moreover, even in the new bone volume (NV) evaluation, the NV value of the first group (Sample 1-2) was approximately 2.0 times higher than that of the fourth group (Comparative Example 3). In addition, it was confirmed that the NV value of the first group (Sample 1-2) was approximately 1.6 times higher than the TV value of the fifth group (Comparative Example 4).

From the above analysis results, it was proven that the osteogenic ability of the bone prepared graft material including whitlockite according to an embodiment of the present application, is considerably higher than that of the hydroxyapatite-based bone graft material, the β-TCP-based bone graft material, or the hydroxyapatite-β-TCP composite graft material.

Staining was used as one of the analysis methods for evaluating osteogenic ability. Here, the osteogenic ability of the control and the experimental groups was evaluated in various ways using various staining analysis methods such as Hematoxylin-Eosin (H&E) staining, Masson's Trichrome staining, and immunohistochemistry.

The nucleus of a cell was stained with hematoxylin, and other cellular structures were stained with eosin, followed by overall evaluation of the entire tissue.

Specifically, after sectioning the paraffin block formed by embedding the decalcified tissue sample with paraffin, deparaffination was performed using an organic solvent, and then the organic solvent residue was removed through ethanol washing. Subsequently, after staining the nucleus by dipping tissue in a hematoxylin solution for approximately 1 to 2 minutes, differentiation and washing were performed using ethanol and running water.

After the hematoxylin staining was completed, the cytoplasm excluding the nucleus was stained by dipping in an eosin solution for 10 to 20 seconds. Afterward, dehydration was performed through sequential precipitation in 50% ethanol (EtOH) and 100% ethanol (EtOH). In addition, the remaining ethanol was removed with xylene.

The stained tissue was encapsulated and dried using an encapsulant and a cover glass. Subsequently, the stained tissue was observed using a microscope.

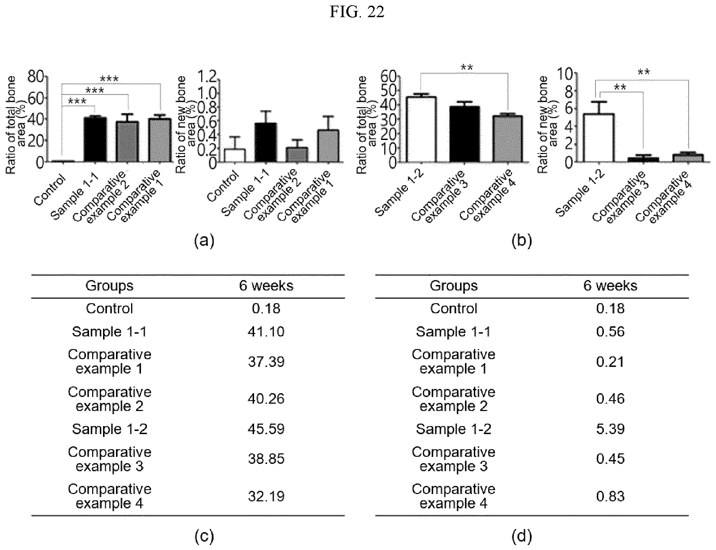

In FIGS. 22, (a) and (b) are graphs illustrating the TV and NV of the control and experimental groups measured through H&E staining. In FIG. 22, (c) is a table illustrating the TV of the control and experimental groups measured through H&E staining. In FIG. 22, (d) is a table illustrating the NV of the control and experimental groups measured through H&E staining. Referring to (a), (b), (c), and (d) of FIG 22, in all experimental groups into which a bone graft material was inserted, it was confirmed that the new bone volume (NV) and total bone volume (TVs) were significantly higher than those of the control into which no bone graft material was inserted. Specifically, at 6 weeks after the implantation of a bone graft material, the total bone area ratio was measured to be approximately 0.18% for the control, whereas the total bone area ratio was measured to be approximately 41.10% for the bone graft material of Sample 1-1, and the total bone area ratio was measured to be 45.49% for the bone graft material of Sample 1-2. In addition, at 6 weeks after the implantation of a bone graft material, the new bone volume (NV) area ratio was measured to be approximately 0.18% for the control, whereas the new bone volume (NV) area ratio was measured to be approximately 0.56% for the bone graft material of Sample 1-1, and the new bone volume (NV) area ratio was measured to be approximately 5.39% for the bone graft material of Sample 1-2.

In addition, in the case of the first group into which the whitlockite-based bone graft material of Sample 1-1 was inserted, it was proven that the new bone volume (NV) was significantly higher than that of the groups into which an same kind bone graft material was inserted. Specifically, the new bone area ratio was measured to be approximately 0.56% for the bone graft material of Sample 1-1, the new bone area ratio was measured to be approximately 0.21% for the bone graft material prepared according to Comparative Example 1, and the new bone area ratio was measured to be approximately 0.46% for the bone graft material prepared according to Comparative Example 2.

In the new bone area ratio evaluation, the new bone area ratio of the first group (Sample 1-1) was approximately 2.6 times higher than that of the second group (Comparative Example 1) into which a hydroxyapatite-based bone graft material was inserted. In addition, it was confirmed that the new bone area ratio of the first group was approximately 1.2 times higher than that of the third group (Comparative Example 2) into which a β-TCP-based bone graft material was inserted.

In addition, in the case of the first group into which the whitlockite-based bone graft material of Sample 1-2 was inserted, it was proven that the new bone area ratio and the total bone area ratio were significantly higher than those of the group into which a β-TCP-HAp composite graft material was inserted. Specifically, the new bone area ratio was measured to be approximately 5.39% for the bone graft material of Sample 1-2, the new bone area ratio was measured to be approximately 0.45% for the bone graft material prepared according to Comparative Example 3, and the new bone area ratio was measured to be approximately 0.83% for the bone graft material prepared according to Comparative Example 4. In addition, the total bone area ratio was measured to be approximately 45.59% for the bone graft material of Sample 1-2, the total bone area ratio was measured to be approximately 38.85% for the bone graft material prepared according to Comparative Example 3, and the total bone area ratio was measured to be approximately 32.19% for the bone graft material prepared according to Comparative Example 4.

In the total bone volume (TV) evaluation, the total bone area ratio of the first group (Sample 1-2) was approximately 1.1 times higher than that of the fourth group (Comparative Example 3). In addition, it was confirmed that the total bone area ratio of the first group was approximately 1.4 times higher than that of the fifth group (Comparative Example 4).

Moreover, even in the new bone volume (NV) evaluation, the new bone area ratio of the first group (Sample 1-2) was approximately 11 times higher than that of the fourth group (Comparative Example 3). In addition, it was confirmed that the new bone area ratio of the first group was approximately 6.5 times higher than that of the fifth group (Comparative Example 4).

From the above analysis results, it was additionally proven that the osteogenic ability of the prepared bone graft material including whitlockite according to an embodiment of the present application is considerably higher than that of the hydroxyapatite-based bone graft material or the β-TCP-based bone graft material.

The degree of bone regeneration (or bone formation) was evaluated through staining of osteoblasts and collagen fibers of connective tissue.

Specifically, after sectioning the paraffin block formed by embedding the decalcified tissue sample with paraffin, deparaffination was performed using an organic solvent, and then the organic solvent residue was removed through ethanol washing. Afterward, the tissue slide was placed in Bouin's solution and mordanted, and then washed with running water. After staining the nucleus with Weigert's HEMA solution for approximately 10 minutes, the slide was neutralized with running water for 10 minutes. Subsequently, staining was performed with Biebrich Scarlet-Acid Fuchsin solution for approximately 10 minutes, washed with running water, and treated with a PMA solution. After washing with running water, sensitization was performed with acetic acid for 10 minutes, and staining was completed by dipping in an aniline blue solution. In addition, the resultant was soaked in running water to remove a dye solution and dried.

The stained tissue was encapsulated and dried using an encapsulant and a cover glass. Subsequently, the stained tissue was observed using a microscope.

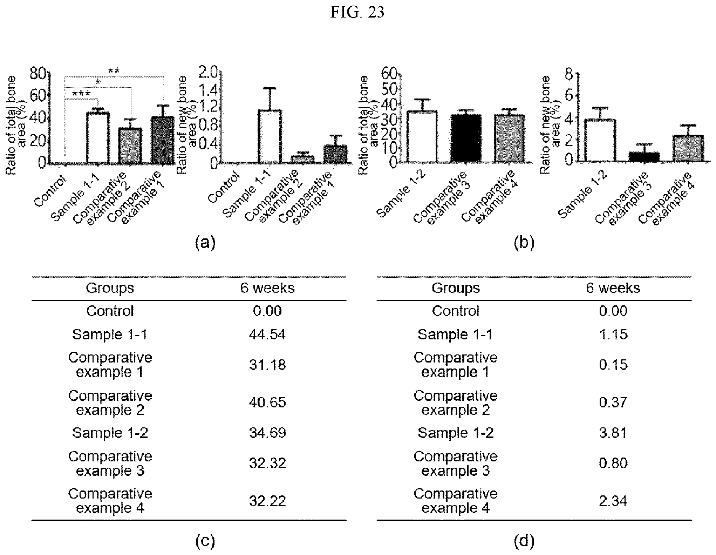

In FIG. 23, (a) and (b) are graphs illustrating the TV and NV of the control and experimental groups measured through Masson's Trichrome staining. In FIG. 23, (c) is a table illustrating the TV of the control and experimental groups measured through Masson's Trichrome staining. In FIG. 23, (d) is a table illustrating the NV of the control and experimental groups measured through Masson's Trichrome staining.

Referring to (a), (b), (c), and (d) of FIG. 23, like FIGS. 21 and 22, in all experimental groups into which a bone graft material was inserted, it was confirmed that the new bone volume (NV) and the total bone volumes (TV) were significantly higher than that of the control into which no bone graft material was inserted. Specifically, at 6 weeks after the implantation of a bone graft material, the total bone area ratio was measured to be approximately 0.00% for the control, whereas the total bone area ratio was measured to be approximately 44.54% for the bone graft material of Sample 1-1, and the total bone area ratio was measured to be 34.60% for the bone graft material of Sample 1-2. In addition, at 6 weeks after the implantation of a bone graft material, the new bone volume (NV) area ratio was measured to be approximately 0.00% for the control, whereas the new bone volume (NV) area ratio was measured to be approximately 1.15% for the bone graft material of Sample 1-1, and the new bone volume (NV) area ratio was measured to be approximately 3.81% for the bone graft material of Sample 1-2.

Particularly, in the case of the first group into which the whitlockite-based bone graft material of Sample 1-1 was inserted, it was proven that the new bone volume (NV) was significantly higher than that of the groups into which a same type of bone graft material was inserted. Specifically, the new bone area ratio was measured to be approximately 1.15% for the bone graft material of Sample 1-1, the new bone area ratio was measured to be approximately 0.15% for the bone graft material prepared according to Comparative Example 1, and the new bone area ratio was measured to be approximately 0.37% for the bone graft material prepared according to Comparative Example 2.

In the new bone volume (NV) evaluation, the new bone area ratio of the first group (Sample 1-1) was approximately 7.8 times higher than that of the second group into which a hydroxyapatite-based bone graft material was inserted (Comparative Example 1). In addition, it was confirmed that the new bone area ratio of the first group was approximately 3 times higher than that of the third group into which a $\beta$-TCP-based bone graft material was inserted (Comparative Example 2).

Additionally, in the case of the first group into which the whitlockite-based bone graft material of Sample 1-2 was inserted, it was proven that the new bone volume (NV) was higher than that of the group into which a $\beta$-TCP-HAp composite graft material was inserted. Specifically, the new bone area ratio was measured to be approximately 3.81% for the bone graft material of Sample 1-2, the new bone area ratio was measured to be approximately 0.80% for the bone graft material prepared according to Comparative Example 3, and the new bone area ratio was measured to be approximately 2.34% for the bone graft material prepared according to Comparative Example 4.

In the new bone volume (NV) evaluation, the new bone area ratio of the first group (Sample 1-2) was approximately 4.7 times higher than that of the fourth group (Comparative Example 3). In addition, it was confirmed that the new bone area ratio of the first group was approximately 1.6 times higher than that of the fifth group (Comparative Example 4).

Moreover, even in the total bone volume (TV) evaluation, it was confirmed that the total bone area ratio of the first group (Sample 1-2) is relatively higher than those of the fourth group (Comparative Example 3) and the fifth group (Comparative Example 4).

From the above analysis results, it was additionally proven that the osteogenic ability, particularly, ability of new bone formation of the prepared bone graft material including whitlockite according to an embodiment of the present application is considerably higher than that of the hydroxyapatite-based bone graft material or the $\beta$-TCP-based bone graft material.

Immunostaining was performed using antibodies against osteocalcin and osteopontin, which are involved in bone matrix organization and deposition and are used as osteogenesis markers.

Specifically, after sectioning the paraffin block formed by embedding the decalcified tissue sample with paraffin, deparaffination was performed using an organic solvent, and then the organic solvent residue was removed through ethanol washing. Next, the reaction of proteins that non-specifically bind to the primary antibodies of proteins to be used for evaluation was inhibited using a blocking solution. Subsequently, the primary antibodies of osteocalcin or osteopotin and secondary antibodies were sequentially reacted. Finally, the proteins in tissue were expressed through a reaction with a detection system and a coloring agent attached to the secondary antibody. The stained tissue was encapsulated and dried using an encapsulant and a cover glass. Subsequently, the stained tissue was observed using a microscope.

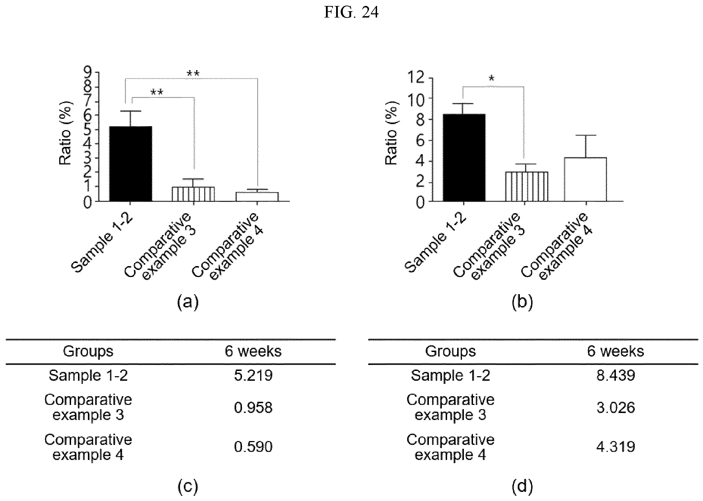

FIG. 24 is a graph and table illustrating the relative proportions of biomarkers associated with osteogenesis measured by immunohistochemistry. Specifically, in FIG. 24, (a) is a graph illustrating the relative proportion of osteocalcin associated with osteogenesis, and (b) of FIG. 24 is a graph illustrating the relative proportion of osteopontin associated with osteogenesis. In FIG. 24, (c) is a table illustrating the relative proportion of osteocalcin associated with osteogenesis, and (d) of FIG. 24 is a table illustrating the relative proportion of osteopontin associated with osteogenesis.

Referring to (a) and (c) of FIG. 24, in the case of the first group into which the whitlockite-based bone graft material of Sample 1-2 was inserted, compared with the group into which a $\beta$-TCP-HAp composite graft material was inserted, it was confirmed that the relative proportion (integral optical density ratio, %) of expressed osteocalcin was high. Specifically, the expression level of osteocalcin was measured to be 5.219% for the bone graft material of Sample 1-2, the expression level of osteocalcin was measured to be 0.958% for the bone graft material prepared according to Comparative Example 3, and the expression level of osteocalcin was measured to be 0.590% for the bone graft material prepared according to Comparative Example 4. Particularly, the relative proportion of the expressed osteocalcin in the first group (Sample 1-2) was 5.4 times higher than that of the fourth group (Comparative Example 3). In addition, the relative proportion of the expressed osteocalcin in the first group was 8.8 times higher than that of the fifth group (Comparative Example 4).

Referring to (b) and (d) of FIG. 24, in the case of the first group into which the whitlockite-based bone graft material of Sample 1-2 was inserted, it was confirmed that the relative proportion (integral optical density ratio, %) of expressed osteopontin was higher than that of the group into which a $\beta$-TCP-HAp composite graft material was inserted. Specifically, the expression level of osteopontin was measured to be 8.439% for the bone graft material of Sample 1-2, the expression level of osteopontin was measured to be 3.026% for the bone graft material prepared according to Comparative Example 3, and the expression level of osteopontin was measured to be 4.319% for the bone graft material prepared according to Comparative Example 4. Particularly, the relative proportion of the expressed osteopontin in the first group (Sample 1-2) was 2.7 times higher than that of the fourth group (Comparative Example 3). In addition, the relative proportion of the expressed osteopontin in the first group was 1.9 times higher than that of the fifth group (Comparative Example 4).

Osteocalcin and osteopontin are biomarkers closely related to bone regeneration, and from the above analysis results, it can be proven that the bone regeneration effect of the prepared bone graft material including whitlockite according to an embodiment of the present invention is much higher than that of the hydroxyapatite-based bone graft material, the β-TCP-based bone graft material, or the hydroxyapatite-β-TCP composite graft material.

Summarizing the above analysis results, it can be confirmed that when the bone graft material according to an embodiment of the present application was inserted into the body, new bone was generated around the insertion site, and the total bone volume increased. Particularly, it was proven that when the bone graft material according to an embodiment of the present application was implanted, more new bone was generated than the hydroxyapatite-based bone graft material, the β-TCP-based bone graft material, or the hydroxyapatite-β-TCP composite graft material. In other words, it was confirmed from the above analysis results that the bone graft material according to an embodiment of the present application exhibited better osteogenic ability than the hydroxyapatite-based bone graft material and the β-TCP-based bone graft material.

Additionally, through immunochemistry for the bone regeneration-related biomarkers, it was proven that when the bone graft material according to an embodiment of the present application was implanted, the expression of the bone regeneration-related biomarkers was relatively more highly induced than the hydroxyapatite-based bone graft material or β-TCP-based bone graft material.

These results are predicted to be due to complex factors, such as a higher specific surface area, higher wettability, and a relatively high compressive strength of the bone graft material according to an embodiment of the present application, compared with the bone graft materials of Comparative Examples.

**Analysis of degree of cell adhesion**

*Method of analyzing degree of cell adhesion*

The cell adhesion ability of the bone graft materials prepared according to Preparation Example and Comparative Examples was confirmed by measuring CCK-8 cell activity using SAOS-2 cells. Specifically, Ctrl (control) was placed in a cell culture plate, 100 mg each of the prepared bone graft materials was applied to $10^5$ cells/mL, and the plate was incubated at 37 °C for 5 hours and then washed with PBS three times to remove unadhered cells. Afterward, the activity of the cells adhered to each bone graft material was measured.

*Analysis results*

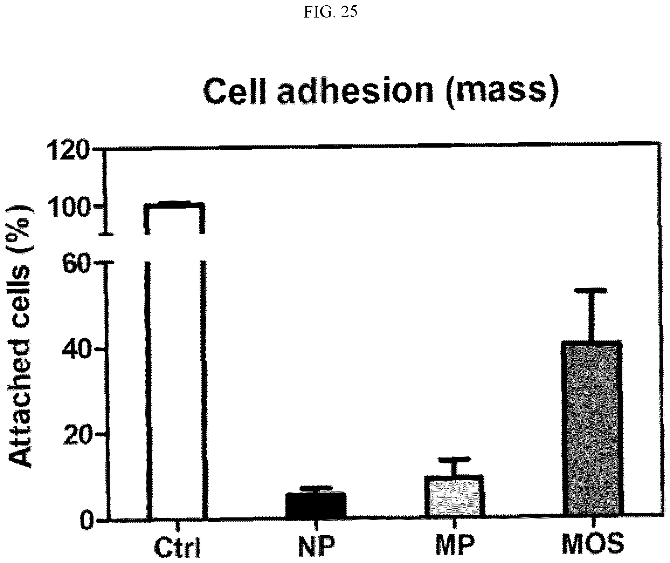

The experimental data for the degree of cell adhesion to the bone graft materials prepared according to Preparation Example and Comparative Examples is shown in FIG. 25. In FIG. 25, NP indicates a group treated with the bone graft material of Comparative Example 5 (whitlockite bone graft material with only nanopores), and MP indicates a group treated with the bone graft material of Comparative Example 6 (whitlockite bone graft material with only micropores). MOS indicates a group treated with the whitlockite bone graft material of Sample 1-4 (bone graft material prepared according to Preparation Example).

Referring to FIG. 25, it can be seen that the bone graft material prepared according to Preparation Example shows a higher degree of cell adhesion than the bone graft material with only nanopores and the bone graft material with only micropores (NP: 5.45%; MP: 9.18%; MOS: 40.14%).

**Comparison of osteogenic differentiation**

The osteogenic differentiation of the bone graft materials prepared according to Preparation Example and Comparative Examples is confirmed by ALP and ARS staining using ADSC cells. Specifically, the prepared bone graft materials is eluted in a differentiation medium at a concentration of 4 g/100 mL at 37 °C for 3 days. After seeding $10^5$ cells in a well culture plate, the degree of initial osteogenic differentiation is confirmed through ALP staining one week after the application of the eluate. Three weeks after the application of the eluate, the degree of late osteogenic differentiation is confirmed through ARS staining.

**Claims**

**1.** Porous bone graft material consisting of whitlockite, comprising:

> nano pores with size of 100nm to 1000nm;
> micro pores with size of 1 μm to 100 μm; and
> macro pores with size of 100 μm to 1000 μm.

2. The bone graft material of Claim 1,
wherein a volume formed by the nano pores with size of 100nm to 1000nm per unit mass of the bone graft material is 0.15 to 0.5ml/g.

3. The bone graft material of Claim 1,
wherein a volume formed by the micro pores with size of 1 $\mu$m to 100 $\mu$m per unit mass of the bone graft material is 0.06 to 0.35ml/g.

4. The bone graft material of Claim 1,
wherein a volume formed by the macro pores with size of 100$\mu$m to 1000$\mu$m per unit mass of the bone graft material is 0.7 to 1.6ml/g.

5. The bone graft material of Claim 1,

wherein a volume formed by the nano pores with size of 100nm to 1000nm per unit mass of the bone graft material is 0.15 to 0.5ml/g,
wherein a volume formed by the micro pores with size of 1 $\mu$m to 100 $\mu$m per unit mass of the bone graft material is 0.06 to 0.35ml/g, and
wherein a volume formed by the macro pores with size of 100$\mu$m to 1000$\mu$m per unit mass of the bone graft material is 0.7 to 1.6ml/g.

6. The bone graft material of Claim 1,

wherein a volume% of the macro pores with size of 100$\mu$m to 1000$\mu$m is 50% to 90%,
wherein the volume% of the macro pores with size of 100$\mu$m to 1000$\mu$m is calculated by following equation:

$$a\ (\%) = 100 \times \frac{\text{volume formed by macropores per unit mass of whitlockite bone graft material}}{\text{volume formed by all pores per unit mass of whitlockite bone graft material}},$$

wherein a is the volume% of the macro pores with size of 100$\mu$m to 1000$\mu$m.

7. The bone graft material of Claim 1,

wherein a volume% of the micro pores with size of 1$\mu$m to 100$\mu$m is 4.5% to 25%,
wherein the volume% of the micro pores with size of 1$\mu$m to 100$\mu$m is calculated by following equation:

$$b\ (\%) = 100 \times \frac{\text{volume formed by micropores per unit mass of whitlockite bone graft material}}{\text{volume formed by all pores per unit mass of whitlockite bone graft material}},$$

wherein b is the volume% of the micro pores with size of 1$\mu$m to 100$\mu$m.

8. The bone graft material of Claim 1,

wherein a volume% of the nano pores with size of 100nm to 1000nm is 8% to 30%,
wherein the volume% of the nano pores with size of 100nm to 1000nm is calculated by following equation:

$$c\ (\%) = 100 \times \frac{\text{volume formed by nanopores per unit mass of whitlockite bone graft material}}{\text{volume formed by all pores per unit mass of whitlockite bone graft material}},$$

wherein c is the volume% of the nano pores with size of 100nm to 1000nm.

9. The bone graft material of Claim 1,

wherein a volume% of the macro pores with size of 100μm to 1000μm is 50% to 90%,
wherein a volume% of the micro pores with size of 1μm to 100μm is 4.5% to 25%, and
wherein a volume% of the nano pores with size of 100nm to 1000nm is 8% to 30%,
wherein each of the volume% of the macro pores with size of 100μm to 1000μm, the volume% of the micro pores with size of 1μm to 100μm, and the volume% of the nano pores with size of 100nm to 1000nm is calculated by the following equation:

$$a\,(\%) = 100 \times \frac{\text{volume formed by macropores per unit mass of whitlockite bone graft material}}{\text{volume formed by all pores per unit mass of whitlockite bone graft material}}$$

$$b\,(\%) = 100 \times \frac{\text{volume formed by micropores per unit mass of whitlockite bone graft material}}{\text{volume formed by all pores per unit mass of whitlockite bone graft material}}$$

$$c\,(\%) = 100 \times \frac{\text{volume formed by nanopores per unit mass of whitlockite bone graft material}}{\text{volume formed by all pores per unit mass of whitlockite bone graft material}} ,$$

wherein a is the volume% of the macro pores with size of 100μm to 1000μm, b is the volume% of the micro pores with size of 1μm to 100μm, and c is the volume% of the nano pores with size of 100nm to 1000nm.

10. The bone graft material of Claim 1,
wherein the bone graft material has porosity of 60% to 95%.

11. The bone graft material of Claim 1,
wherein the bone graft material has compressive strength of 500KPa to 900KPa.

12. The bone graft material of Claim 1,
wherein the bone graft material has specific surface area of $5m^2/g$ to $6m^2/g$.

13. The bone graft material of Claim 12,
wherein the specific surface area is analyzed by Brunauer-Emmeet-Teller method.

14. The bone graft material of Claim 1,
wherein the bone graft material has a water absorption property of 2.0g or more per unit mass.

FIG. 1

FIG. 2

FIG. 3

FIG. 4

100X

Comparative
example 1

Comparative
example 3

Comparative
example 2

Comparative
example 4

500X

Comparative
example 1

Comparative
example 3

Comparative
example 2

Comparative
example 4

FIG. 5

FIG. 6

FIG. 7

FIG.  8

| | Sample 1-2(1) | Sample 1-2(2) | Sample 1-3 | Sample 1-4 |
|---|---|---|---|---|
| Porosity | 69.75% | 86.43% | 87.01% | 78.8% |

FIG. 9

FIG. 10

Sample 1-3

FIG. 11

Sample 1-4

FIG. 12

|  | Comparative example 3 | Comparative example 4 | Comparative example 5 | Comparative example 6 |
|---|---|---|---|---|
| Porosity | 66.4% | 82.9% | 39.83% | 42.12% |

FIG. 13

(b)

(c)

**Comparative example 5**

**Comparative example 6**

FIG. 14

FIG. 15

| | Sample 1-2 | Comparative example 3 | Comparative example 4 |
|---|---|---|---|
| Specifie surface area $(m^2/g)$ | 5.62 | 0.28 | 0.04 |

FIG. 16

FIG. 17

Results for compressive strength

FIG. 18

|  | Sample 1-2 | Comparative example 3 | Comparative example 4 |
|---|---|---|---|
| Loss of weight (%) | 0.02 | 0.56 | 2.83 |

FIG. 19

FIG. 20

Control     Sample 1-1     Comparative example 2     Comparative example 1

Sample 1-2     Comparative example 3     Comparative example 4

FIG. 21

(a)                                                           (b)

| Groups | 6 weeks |
|---|---|
| Control | 0.08 |
| Sample 1-1 | 51.35 |
| Comparative example 1 | 32.67 |
| Comparative example 2 | 42.00 |
| Sample 1-2 | 19.99 |
| Comparative example 3 | 10.88 |
| Comparative example 4 | 9.50 |

(c)

| Groups | 6 weeks |
|---|---|
| Control | 0.08 |
| Sample 1-1 | 17.60 |
| Comparative example 1 | 6.20 |
| Comparative example 2 | 11.93 |
| Sample 1-2 | 6.50 |
| Comparative example 3 | 3.25 |
| Comparative example 4 | 4.01 |

(d)

FIG. 22

(a)

(b)

| Groups | 6 weeks |
|---|---|
| Control | 0.18 |
| Sample 1-1 | 41.10 |
| Comparative example 1 | 37.39 |
| Comparative example 2 | 40.26 |
| Sample 1-2 | 45.59 |
| Comparative example 3 | 38.85 |
| Comparative example 4 | 32.19 |

(c)

| Groups | 6 weeks |
|---|---|
| Control | 0.18 |
| Sample 1-1 | 0.56 |
| Comparative example 1 | 0.21 |
| Comparative example 2 | 0.46 |
| Sample 1-2 | 5.39 |
| Comparative example 3 | 0.45 |
| Comparative example 4 | 0.83 |

(d)

FIG. 23

(a)                    (b)

| Groups | 6 weeks |
|---|---|
| Control | 0.00 |
| Sample 1-1 | 44.54 |
| Comparative example 1 | 31.18 |
| Comparative example 2 | 40.65 |
| Sample 1-2 | 34.69 |
| Comparative example 3 | 32.32 |
| Comparative example 4 | 32.22 |

(c)

| Groups | 6 weeks |
|---|---|
| Control | 0.00 |
| Sample 1-1 | 1.15 |
| Comparative example 1 | 0.15 |
| Comparative example 2 | 0.37 |
| Sample 1-2 | 3.81 |
| Comparative example 3 | 0.80 |
| Comparative example 4 | 2.34 |

(d)

FIG. 24

(a)

(b)

| Groups | 6 weeks |
|---|---|
| Sample 1-2 | 5.219 |
| Comparative example 3 | 0.958 |
| Comparative example 4 | 0.590 |

(c)

| Groups | 6 weeks |
|---|---|
| Sample 1-2 | 8.439 |
| Comparative example 3 | 3.026 |
| Comparative example 4 | 4.319 |

(d)

FIG. 25

Cell adhesion (mass)

## INTERNATIONAL SEARCH REPORT

| International application No. |
| --- |
| **PCT/KR2022/012954** |

### A. CLASSIFICATION OF SUBJECT MATTER

**A61L 27/12**(2006.01)i; **A61L 27/56**(2006.01)i; **A61F 2/28**(2006.01)i

According to International Patent Classification (IPC) or to both national classification and IPC

### B. FIELDS SEARCHED

Minimum documentation searched (classification system followed by classification symbols)

A61L 27/12(2006.01); A61F 2/02(2006.01); A61F 2/28(2006.01); A61L 27/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 골이식재(bone graft material), 나노 기공(nano pore), 마이크로 기공(micro pore), 매크로 기공(macro pore), 기공 부피(pore volume), 압축강도(compressive strength), 비표면적(specific surface area)

### C. DOCUMENTS CONSIDERED TO BE RELEVANT

| Category* | Citation of document, with indication, where appropriate, of the relevant passages | Relevant to claim No. |
| --- | --- | --- |
| Y | LEE, Won-Bum et al. Whitlockite granules on bone regeneration in defect of rat calvaria. ACS Applied Bio Materials. 2020, vol. 3, pp. 7762-7768.<br>See pages 7763-7765. | 1-14 |
| Y | US 2009-0157182 A1 (KOBLISH, Antony et al.) 18 June 2009 (2009-06-18)<br>See paragraphs [0012], [0063], [0094] and [0102]; and claims 1, 3 and 14. | 1-14 |
| A | WINNETT, James et al. Porous 3D bioscaffolds by adaptive foam reticulation. Bioinspired, Biomimetic and Nanobiomaterials. 11 January 2014, vol. 4, pp. 4-14.<br>See entire document. | 1-14 |
| A | OH, Daniel S. et al. Bone marrow absorption and retention properties of engineered scaffolds with micro-channels and nano-pores for tissue engineering: a proof of concept. Ceramics International. 2013, vol. 39, pp. 8401-8410.<br>See entire document. | 1-14 |
| A | JP 2000-093504 A (ISOTIS BV) 04 April 2000 (2000-04-04)<br>See entire document. | 1-14 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **24 November 2022** | **28 November 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

| Patent document<br>cited in search report | Publication date<br>(day/month/year) | Patent family member(s) | Publication date<br>(day/month/year) |
|---|---|---|---|
| US   2009-0157182   A1 | 18 June 2009 | US   2005-0169893   A1 | 04 August 2005 |
| | | US   2005-0169956   A1 | 04 August 2005 |
| | | US   2005-0214340   A1 | 29 September 2005 |
| | | US   7189263   B2 | 13 March 2007 |
| | | US   7531004   B2 | 12 May 2009 |
| | | US   7534451   B2 | 19 May 2009 |
| | | US   8287915   B2 | 16 October 2012 |
| | | WO   2005-074614   A2 | 18 August 2005 |
| JP   2000-093504   A | 04 April 2000 | US   2002-0035402   A1 | 21 March 2002 |
| | | US   6511510   B1 | 28 January 2003 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021080348 A

**Non-patent literature cited in the description**

- **JANG, HAE LIN et al.** Phase transformation from hydroxyapatite to the secondary bone mineral, whitlockite. *Journal of Materials Chemistry,* 2015, vol. 3 (7), 1342-1349